(19) Europäisches Patentamt
European Patent Office
Office européen des brevets

(11) EP 3 932 981 A1

(12) # EUROPEAN PATENT APPLICATION
published in accordance with Art. 153(4) EPC

(43) Date of publication:
**05.01.2022 Bulletin 2022/01**

(21) Application number: **20763672.1**

(22) Date of filing: **25.02.2020**

(51) Int Cl.:
***C08K 5/101*** *(2006.01)* ***C08K 5/13*** *(2006.01)*
***C08K 5/3472*** *(2006.01)* ***C08K 5/3477*** *(2006.01)*
***C08K 5/521*** *(2006.01)* ***C08L 69/00*** *(2006.01)*

(86) International application number:
**PCT/JP2020/007514**

(87) International publication number:
**WO 2020/175487 (03.09.2020 Gazette 2020/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.02.2019 JP 2019032917**
**26.02.2019 JP 2019032878**

(71) Applicants:
- **Teijin Limited**
**Osaka 530-0005 (JP)**
- **Miyoshi Oil & Fat Co., Ltd.**
**Tokyo 124-8510 (JP)**

(72) Inventors:
- **OHIRA, Yoji**
**Osaka-shi, Osaka 530-0005 (JP)**
- **SHUTO, Hiroshi**
**Osaka-shi, Osaka 530-0005 (JP)**
- **TANABE, Seiichi**
**Osaka-shi, Osaka 530-0005 (JP)**
- **ISSHIKI, Hideki**
**Osaka-shi, Osaka 530-0005 (JP)**
- **KAWAI, Koji**
**Tokyo 124-8510 (JP)**
- **KANEKO, Kotaro**
**Tokyo 124-8510 (JP)**
- **KANEKO, Nobuhiro**
**Tokyo 124-8510 (JP)**
- **YASHITA, Akira**
**Tokyo 124-8510 (JP)**
- **NAKAMURA, Daisuke**
**Tokyo 124-8510 (JP)**

(74) Representative: **Beckmann, Claus**
**Kraus & Weisert**
**Patentanwälte PartGmbB**
**Thomas-Wimmer-Ring 15**
**80539 München (DE)**

(54) **POLYCARBONATE RESIN COMPOSITION**

(57) Provided is a polycarbonate resin composition having excellent impact strength, molding residence stability, and dry heat resistance and that maintains high total light transmittance while suppressing light transmittance in a specific ultraviolet region, that is, harmful wavelengths near 420 nm. The polycarbonate resin composition of the present disclosure includes 0.1-1.5 mass part of an additive (B) per 100 mass parts of a polycarbonate resin (A) having a viscosity average molecular weight of 21,000-26,000 and includes 0.2 mass part or more of an ultraviolet absorber (B1) having a benzotriazole skeleton represented by formula 1 as the additive (B): In formula 1, $R^1$-$R^5$ each independently represent any of a hydrogen atom, a C1-20 hydrocarbon group, and a hydroxyl group, and the hydrocarbon group(s) may include an oxygen-containing group; $R^6$-$R^9$ each independently represent any of a hydrogen atom or a sulfur-containing group represented by R-S-, and at least one of $R^6$-$R^9$ is a sulfur-containing group represented by R-S-; and R is a C1-24 hydrocarbon group or a C6-24 aromatic group in which a hydrogen atom may be substituted by a C1-18 alkyl group.



Printed by Jouve, 75001 PARIS (FR)

## Description

FIELD

The present disclosure relates to a polycarbonate resin composition which maintains a high total light transmittance while reducing the transmittance of light in a specific ultraviolet region, i.e. light having a wavelength of about 420 nm which is considered as harmful light, and exhibits excellent impact resistance and molding residence stability.

BACKGROUND

Molded articles produced from synthetic resins (plastics) are less likely to be cracked and can be more easily colored and molded in various manners as compared to glass materials; therefore, glass materials have been increasingly replaced by such molded articles. Synthetic resins have come into use in place of glass materials also in, for example, optical molded articles such as spectacle lenses, camera lenses, front panels of various displays, and light source covers.
Synthetic resin molded articles, however, are likely to be deteriorated by exposure to sunlight or ultraviolet radiation over a prolonged period and, for example, deterioration such as yellowing are inevitable. In order to inhibit deterioration such as discoloration, ultraviolet absorbers are incorporated into synthetic resin molded articles for improvement of the light resistance.
In addition, it has been recently studied to provide a synthetic resin molded article with an ultraviolet absorption capacity for eye protection. Adverse effects of ultraviolet radiation on the eyes have been previously pointed out. Further, there is a concern that, for example, natural light and blue light emitted from a liquid crystal display of an office device, a display of a portable device such as a smartphone or a mobile phone, or the like may have an effect on the eyes, such as eye fatigue or pain. For example, it has been indicated that a prolonged exposure of the eyes to blue light causes asthenopia and oxidative stress due to the generation of reactive oxygen species, particularly excess singlet oxygen. It is known that the generation of singlet oxygen is facilitated by a short-wavelength blue light which has a high energy among ultraviolet rays and visible light.
In the retina, a waste called lipofuscin is accumulated in the retinal pigment epithelium with aging, and this lipofuscin is believed to act as a photosensitizer to generate singlet oxygen. Lipofuscin has a property of exhibiting an increased absorption at a shorter wavelength over a range of visible light to ultraviolet region. Meanwhile, lutein is known as a substance that reduces the oxidative stress caused by singlet oxygen. Lutein, which exists in the retina, is degraded by light in the ultraviolet to blue region.
Accordingly, in order to suppress the generation of singlet oxygen by lipofuscin and to inhibit the deterioration of lutein that reduces oxidative stress, it is extremely effective to cut off a wavelength of 400 to 420 nm, which is in a wavelength range where the light absorption characteristics of lutein and lipofuscin overlap, before it reaches the retina.
Further, recent studies indicate that an exposure of retinal tissue to a light having a short wavelength of 411 nm imposes a stronger oxidative stress on the neuronal retinal cells than an exposure to a light having a wavelength of 470 nm, leading to observation of signs of cell death and inducing strain in the structure of retinal tissue, and this is believed as one of the factors that cause age-related macular degeneration to advance.
Moreover, it has been pointed out that the generation of reactive oxygen species causing cortical cataract, as well as DNA damage and death of lens epithelial cells, are initiated by irradiation with a light having a wavelength of 400 to 420 nm; therefore, it is extremely important for the maintenance of eye health to block short-wavelength light of 400 to 420 nm that can potentially trigger an ocular tissue disorder. In other words, in order to keep the eyes healthy, it is extremely important to reduce the transmittance of light having a wavelength of about 420 nm.
PTLs 1 and 2 propose the use of an ultraviolet absorber that imparts a plastic molded article with a capacity of absorbing light having a wavelength of 400 to 420 nm.
PTLs 3 and 4 propose plastic lenses which exhibit a reduced absorption of light having a wavelength of 420 nm or longer while sufficiently and efficiently absorbing light having a wavelength of 400 to 420 nm, and have an excellent outer appearance with limited effect of harmful light and reduced yellowing.

[CITATION LIST]

[PATENT LITERATURE]

[PTL 1] Japanese Patent No. 5620033
[PTL 2] Japanese Patent No. 4334633
[PTL 3] WO 2016/021664

[PTL 4] WO 2016/174788

SUMMARY

[TECHNICAL PROBLEM]

PTL 1 proposes a technology of using 2-(2-hydroxy-3-*tert*-butyl-5-methylphenyl)-chlorobenzotriazole as an ultraviolet absorber and combining it with a resin material such as an episulfide resin. Further, PTL 2 proposes a technology of incorporating a specific benzotriazole-based ultraviolet absorber.

However, in order to allow a resin to sufficiently absorb light having a wavelength of about 400 to 420 nm by adding thereto the benzotriazole-based ultraviolet absorber disclosed in PTLs 1 and 2, it is necessary to add the ultraviolet absorber in a large amount due to its low absorption efficiency in this wavelength range and, at the same time, there is a problem that the ultraviolet absorber causes yellowing of a molded article since the ultraviolet absorber also absorbs light having a wavelength longer than about 420 nm in a large amount because of its optical properties.

PTLs 3 and 4 propose transparent plastic molded articles having a reduced transmittance at a wavelength of 420 nm. However, the plastic molded articles such as plastic lenses that are concretely presented in these proposals are molded articles composed of a thermosetting resin having insufficient impact resistance.

Generally, polycarbonate resins are said to have excellent mechanical properties in terms of impact resistance and the like as compared to other thermoplastic resins and thermosetting resins. However, when various additives are used in combination with a polycarbonate resin to provide various functions other than impact resistance, depending on the type and the amount of the additives, there are often problems that the mechanical properties of a molded product are deteriorated, or that the thermal stability in molding is deteriorated, resulting in discoloration of the resulting molded article and deterioration of its physical property.

As described above, transparent plastic molded articles having a reduced transmittance at a wavelength of about 420 nm, which is considered to be harmful, have been proposed; however, no specific proposal has been made with regard to obtaining a polycarbonate resin composition which has excellent impact resistance while reducing the transmittance of light having a wavelength of about 420 nm. In addition, there has been a strong demand for a polycarbonate resin composition which not only reduces the transmittance of light having a wavelength of about 420 nm and has excellent impact resistance, but also has a high total light transmittance and exhibits excellent molding residence stability and dry heat resistance during molding and extrusion.

Accordingly, an object of the present disclosure is to provide a polycarbonate resin composition which maintains a high total light transmittance while reducing the transmittance of light in a specific ultraviolet region, i.e. light having a wavelength of about 420 nm which is considered as harmful light, and exhibits excellent impact resistance and molding residence stability, preferably excellent dry heat resistance as well.

[SOLUTION TO PROBLEM]

<Aspect 1>

A polycarbonate resin composition, containing, with respect to 100 parts by mass of a polycarbonate resin (A) having a viscosity-average molecular weight of 21,000 to 26,000: 0.1 to 1.5 parts by mass of an additive (B); and not less than 0.1 parts by mass of an ultraviolet absorber (B1) having a benzotriazole skeleton represented by the following Formula 1 as the additive (B):

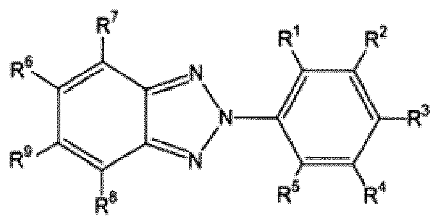

Formula 1

wherein,

$R^1$ to $R^5$ each independently represent any of a hydrogen atom, a hydrocarbon group having 1 to 20 carbon atoms, and a hydroxy group, which hydrocarbon group optionally contains an oxygen-containing group; and

$R^6$ to $R^9$ each independently represent any of a hydrogen atom and a sulfur-containing group represented by R-S-, at least one of which $R^6$ to $R^9$ is a sulfur-containing group represented by R-S- and which R is a hydrocarbon group

having 1 to 24 carbon atoms, or an aromatic group having 6 to 24 carbon atoms in which a hydrogen atom is optionally substituted with an alkyl group having 1 to 18 carbon atoms.

<Aspect 2>

The composition according to mode 1, wherein R of the sulfur-containing group represented by R-S- in the ultraviolet absorber (B1) is a hydrocarbon group having 1 to 24 carbon atoms.

<Aspect 3>

The composition according to mode 2, containing 0.2 to 1.5 parts by mass of the additive (B), and not less than 0.2 parts by mass of the ultraviolet absorber (B1), with respect to 100 parts by mass of the polycarbonate resin (A).

<Aspect 4>

The composition according to mode 2 or 3, wherein R of the sulfur-containing group represented by R-S- in the ultraviolet absorber (B1) represents an alkyl group having 1 to 24 carbon atoms.

<Aspect 5>

The composition according to mode 1, wherein R of the sulfur-containing group represented by R-S- in the ultraviolet absorber (B1) is an aromatic group having 6 to 24 carbon atoms in which a hydrogen atom is optionally substituted with an alkyl group having 1 to 18 carbon atoms.

<Aspect 6>

The composition according to mode 5, wherein R of the sulfur-containing group represented by R-S- in the ultraviolet absorber (B1) represents a phenyl residue.

<Aspect 7>

The composition according to any one of modes 1 to 6, wherein the sulfur-containing group represented by R-S- in the ultraviolet absorber (B1) is bound to $R^6$ or $R^9$.

<Aspect 8>

The composition according to any one of modes 1 to 7, wherein the hydrocarbon group represented by $R^1$ to $R^5$ in the ultraviolet absorber (B1) is an alkyl group having 1 to 12 carbon atoms.

<Aspect 9>

The composition according to any one of modes 1 to 8, wherein the hydrocarbon group represented by $R^1$ to $R^5$ in the ultraviolet absorber (B1) is an alkyl group having 1 to 4 carbon atoms.

<Aspect 10>

The composition according to any one of modes 1 to 8, wherein at least one of $R^1$ to $R^5$ in the ultraviolet absorber (B1) is a methyl group.

<Aspect 11>

The composition according to any one of modes 1 to 8, wherein at least one of $R^1$ to $R^5$ in the ultraviolet absorber (B1) is a methyl group, and at least one of the remaining hydrocarbon groups of $R^1$ to $R^5$ is a butyl group.

<Aspect 12>

The composition according to any one of modes 1 to 11, containing 0.1 to 1.4 parts by mass of the ultraviolet absorber (B1) with respect to 100 parts by mass of the polycarbonate resin (A).

<Aspect 13>

The composition according to any one of modes 1 to 12, containing 0.01 to 0.1 parts by mass of a phosphorus-based heat stabilizer (B2) as the additive (B) with respect to 100 parts by mass of the polycarbonate resin (A).

<Aspect 14>

The composition according to any one of modes 1 to 13, containing 0.03 to 0.5 parts by mass of a fatty acid ester-based mold release agent (B3) as the additive (B) with respect to 100 parts by mass of the polycarbonate resin (A).

<Aspect 15>

The composition according to any one of modes 1 to 14, containing 0.01 to 0.30 parts by mass of a hindered phenol-based antioxidant (B4) as the additive (B) with respect to 100 parts by mass of the polycarbonate resin (A).

<Aspect 16>

The composition according to any one of modes 1 to 15, containing 0.001 to 0.02 parts by mass of an epoxy group-containing compound (B5) as the additive (B) with respect to 100 parts by mass of the polycarbonate resin (A).

<Aspect 17>

The composition according to any one of modes 1 to 16, containing 0.05 to 0.3 parts by mass of an ultraviolet absorber (B6) other than the ultraviolet absorber (B1) as the additive (B) with respect to 100 parts by mass of the polycarbonate resin (A).

<Aspect 18>

The composition according to any one of modes 1 to 17, which is used as a molding material of a hot-melt-molded article.

<Aspect 19>

The composition according to any one of modes 1 to 17, which is used as a molding material of a spectacle lens molded article.

[ADVANTAGEIOUS EFFECTS OF INVENTION]

According to the present disclosure, a polycarbonate resin composition which maintains a high total light transmittance while reducing the transmittance of light in a specific ultraviolet region, i.e. light having a wavelength of about 420 nm which is considered as harmful light, and exhibits excellent impact resistance, molding residence stability and dry heat resistance can be provided.

Further, according to the present disclosure, a polycarbonate resin composition which can be used as a molding material for molding various heat-melt-molded articles, for example, front panels of various displays of personal computers, car navigation systems and the like, front panels and covers of light emitters, light source covers, multi-purpose sheets (e.g., injection-molded flat plates, and extrusion-molded flat plats and films), and spectacle lenses, can be provided.

DESCRIPTION OF EMBODIMENTS

Embodiments of the present disclosure will now be described in detail. The present disclosure, however, is not restricted to the below-described embodiments, and can be carried out with various modifications within the gist of the invention.

The polycarbonate resin composition according to one embodiment of the present disclosure is a polycarbonate resin composition which contains, with respect to 100 parts by mass of a polycarbonate resin (A) having a viscosity-average molecular weight of 21,000 to 26,000: 0.2 to 1.5 parts by mass of an additive (B); and not less than 0.1 parts by mass of an ultraviolet absorber (B1) having a benzotriazole skeleton represented by the following Formula 1 as the additive (B):

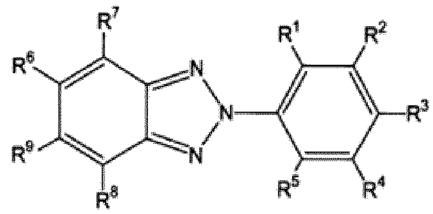

Formula 1

wherein,

- $R^1$ to $R^5$ each independently represent any of a hydrogen atom, a hydrocarbon group having 1 to 20 carbon atoms, and a hydroxy group, which hydrocarbon group optionally contains an oxygen-containing group; and
- $R^6$ to $R^9$ each independently represent any of a hydrogen atom and a sulfur-containing group represented by R-S-, at least one of which $R^6$ to $R^9$ is a sulfur-containing group represented by R-S-, and which R is a hydrocarbon group having 1 to 24 carbon atoms, or an aromatic group having 6 to 24 carbon atoms in which a hydrogen atom is optionally substituted with an alkyl group having 1 to 18 carbon atoms.

The polycarbonate resin composition of the present disclosure maintains a high total light transmittance while reducing the transmittance of light in a specific ultraviolet region, i.e. light having a wavelength of about 420 nm which is considered as harmful light, and exhibits excellent impact resistance and molding residence stability. Particularly, in a mode in which R in Formula 1 is the above-described aromatic group, the polycarbonate resin composition of the present disclosure further has excellent dry heat resistance. Although the polycarbonate resin composition of the present disclosure is not limited by a principle, the action principle thereof is believed to be as follows.

The polycarbonate resin composition of the present disclosure contains a polycarbonate resin having a specific viscosity-average molecular weight, and a specific ultraviolet absorber represented by the above-described Formula 1. It is believed that the use of this ultraviolet absorber enables to maintain a high total light transmittance while sufficiently reducing the transmittance of light having a wavelength of about 420 nm, even in a smaller amount as compared to a conventional benzotriazole-based ultraviolet absorber.

In the ultraviolet absorber, particularly, at least one of $R^6$ to $R^9$ in Formula 1 has a specific sulfur-containing group represented by R-S-. It is believed that, since the ultraviolet absorber has such a specific sulfur-containing group, for example, the heat resistance and the compatibility with a polycarbonate resin are improved, and an effect of inhibiting a reduction in the impact resistance can be obtained even when the ultraviolet absorber is added in a large amount.

Further, a polycarbonate resin composition containing a general ultraviolet absorber is likely to cause clogging of, for example, a vacuum vent pipe connected to an extruder or molding machine, or a volatile matter trapping device arranged in the middle of a vent pipe since, when such a polycarbonate resin composition is extruded or molded, the ultraviolet absorber contained therein itself is easily volatilized and solidified. In other words, in order to allow sufficient ultraviolet absorption capacity to be exerted by using a conventional ultraviolet absorber, it is necessary to add the ultraviolet absorber in a large amount, and this is believed to be a factor that deteriorates the impact resistance and the like of a molded article obtained from a polycarbonate resin composition. On the other hand, as compared to a conventional ultraviolet absorber, the specific ultraviolet absorber of the present disclosure has a higher molecular weight and superior heat resistance and compatibility with a polycarbonate resin and is less likely to be volatilized from a composition; therefore, it is believed that the specific ultraviolet absorber is capable of exerting sufficient ultraviolet absorption capacity in a small amount, without causing a reduction in the impact resistance and the like of a molded article. Moreover, the ultraviolet absorber of the present disclosure can alleviate the problem of clogging a vacuum vent pipe and the like since, as compared to a conventional ultraviolet absorber, the ultraviolet absorber of the present disclosure is less likely to be volatilized and solidification and crystallization thereof is less likely to progress during a molding process that involves heating and melting, such as extrusion.

«Polycarbonate Resin Composition»

The polycarbonate resin composition of the present disclosure is excellent in various performance.

<Light Blocking Performance at 420 nm or Shorter>

The polycarbonate resin composition of the present disclosure can block light of about 420 nm. For example, in the use of a 2 mm-thick sheet obtained from the polycarbonate resin composition, the spectral transmittance of 420-nm light of the sheet can be 70% or lower, lower than 70%, 65% or lower, or 60% or lower. A lower limit value thereof is not particularly restricted; however, it may be defined to be, for example, 0.1% or higher, 0.5% or higher, 1% or higher,

3% or higher, 5% or higher, 7% or higher, or 10% or higher.

The polycarbonate resin composition of the present disclosure can also block light of about 400 nm. For example, in the use of a 2 mm-thick sheet obtained from the polycarbonate resin composition, the spectral transmittance of 400-nm light of the sheet can be 1% or lower, lower than 1%, 0.9% or lower, or 0.8% or lower. A lower limit value thereof is not particularly restricted; however, it may be defined to be, for example, 0% or higher, higher than 0%, or 0.1% or higher.

<Total Light Transmittance>

The polycarbonate resin composition of the present disclosure has excellent transparency. For example, in the use of a 2 mm-thick sheet obtained from the polycarbonate resin composition, the total light transmittance of the sheet can be 87% or higher, higher than 87%, 88% or higher, or 89% or higher. An upper limit value thereof is not particularly restricted; however, it may be defined to be, for example, lower than 100%, 99% or lower, or 98% or lower.

<Impact Resistance>

The polycarbonate resin composition of the present disclosure has excellent impact resistance. For example, a molded article obtained from the polycarbonate resin composition can achieve an impact strength of higher than 55 $kJ/m^2$, 60 $kJ/m^2$ or higher, 70 $kJ/m^2$ or higher, or 80 $kJ/m^2$ or higher in the below-described Charpy impact strength test. An upper limit value thereof is not particularly restricted; however, it may be defined to be, for example, 150 $kJ/m^2$ or lower, 120 $kJ/m^2$ or lower, or 100 $kJ/m^2$ or lower.

<Heat Resistance>

The polycarbonate resin composition of the present disclosure has excellent heat resistance. The term "excellent heat resistance" is intended to mean that deformation of a molded article under a high temperature is small, and the heat resistance can be evaluated by, for example, the below-described test of heat deflection temperature under load. In the test of heat deflection temperature under load, a molded article obtained from the polycarbonate resin composition can achieve a heat deflection temperature of higher than 124°C, 125°C or higher, or 126°C or higher. An upper limit value thereof is not particularly restricted; however, it may be defined to be, for example, 135°C or lower, 132°C or lower, or 130°C or lower.

<Molding Residence Stability>

The polycarbonate resin composition of the present disclosure has excellent molding residence stability. For example, a molded article obtained from the polycarbonate resin composition can achieve a color difference ($\Delta E$) of 0.75 or less, less than 0.75, 0.72 or less, or 0.70 or less in below-described molding residence test. A lower limit value thereof is not particularly restricted; however, it may be defined to be, for example, 0.40 or more, 0.45 or more, or 0.50 or more.

<Dry Heat Resistance>

In a specific mode, the polycarbonate resin composition of the present disclosure has excellent dry heat resistance. The term "excellent dry heat resistance" is intended to mean that, when a molded article is left to stand under a high temperature for a prolonged period, the hue change and deterioration of the molded article are limited. For example, a molded article obtained from the polycarbonate resin composition can achieve a yellowing degree ($\Delta YI$) of 3.0 or lower, lower than 3.0, 2.5 or lower, or 2.0 or lower, in the below-described dry heat resistance test. A lower limit value thereof is not particularly restricted; however, it may be defined to be, for example, 0.1 or higher, 0.3 or higher, or 0.5 or higher.

<Polycarbonate Resin (A)>

A polycarbonate resin that can be incorporated into the polycarbonate resin composition of the present disclosure is not particularly restricted as long as it has a viscosity-average molecular weight of 21,000 to 26,000.

(Viscosity-Average Molecular Weight)

From the standpoints of impact resistance, melt fluidity, moldability and the like, the viscosity-average molecular weight is more preferably 21,500 to 25,000, particularly preferably 22,000 to 24,000.

The viscosity-average molecular weight (M) of a polycarbonate resin is calculated from the following Equations 2 and 3 using a value of specific viscosity (ηsp) that is measured by an Ostwald viscometer for a solution obtained by dissolving 0.7 g of the polycarbonate resin in 100 ml of methylene chloride at 20°C.

$$\eta sp/c = [\eta] + 0.45 \times [\eta]^2 c \quad \text{Equation 2}$$

$$[\eta] = 1.23 \times 10^{-4} M^{0.83} \quad \text{Equation 3}$$

wherein, [η] is an intrinsic viscosity, and c is 0.7

As the polycarbonate resin in the composition of the present disclosure, for example, an aromatic polycarbonate resin obtained by a reaction between a dihydric phenol and a carbonate precursor can be used.

(Dihydric Phenol)

Specific examples of the dihydric phenol include: bis(hydroxyaryl)alkanes, such as 2,2-bis(4-hydroxyphenyl)propane (hereinafter, may be referred to as "bisphenol A"), bis(4-hydroxyphenyl)methane, 1,1-bis(4-hydroxyphenyl)ethane, 2,2-bis(4-hydroxyphenyl)butane, 2,2-bis(4-hydroxyphenyl)octane, 2,2-bis(4-hydroxyphenyl)phenylmethane, 2,2-bis(4-hydroxy-3-methylphenyl)propane, 1,1-bis(4-hydroxy-3-*tert*-butylphenyl)propane, 2,2-bis(4-hydroxy-3-bromophenyl)propane, 2,2-bis(4-hydroxy-3,5-dibromophenyl)propane, and 2,2-bis(4-hydroxy-3,5-dichlorophenyl)propane; bis(hydroxyphenyl)cycloalkanes, such as 1,1-bis(hydroxyphenyl)cyclopentane and 1,1-bis(hydroxyphenyl)cyclohexane; dihydroxyaryl ethers, such as 4,4'-dihydroxydiphenyl ether and 4,4'-dihydroxy-3,3'-dimethyldiphenyl ether; dihydroxydiaryl sulfides, such as 4,4'-dihydroxydiphenyl sulfide and 4,4'-dihydroxy-3,3'-dimethyldiphenyl sulfide; dihydroxydiaryl sulfoxides, such as 4,4'-dihydroxydiphenyl sulfoxide and 4,4'-dihydroxy-3,3'-dimethyldiphenyl sulfoxide; and dihydroxydiaryl sulfones, such as 4,4'-dihydroxydiphenyl sulfone and 4,4'-dihydroxy-3,3'-dimethyldiphenyl sulfone. These dihydric phenols may be used singly, or in combination of two or more thereof.

Among the above-exemplified dihydric phenols, 2,2-bis(4-hydroxyphenyl)propane (bisphenol A) is preferred from the standpoints of impact resistance and the like. The ratio of bisphenol A in all dihydric phenol components is preferably 50% by mole or higher, 60% by mole or higher, 70% by mole or higher, or 80% by mole or higher, and it is more preferably 90% by mole or higher, 95% by mole or higher, or 100%.

(Method of Producing Polycarbonate Resin)

A method of producing a polycarbonate resin is not particularly restricted, and a polycarbonate resin can be produced by, for example, the following method.

For example, a solution method using phosgene as a carbonate precursor can be employed. In this method, usually, a dihydric phenol component and phosgene are allowed to react in the presence of an acid binder and an organic solvent.

As the acid binder, for example, an alkali metal hydroxide such as sodium hydroxide or potassium hydroxide, or an amine compound such as pyridine is used. As the organic solvent, for example, a halogenated hydrocarbon such as methylene chloride or chlorobenzene is used.

In addition, as desired, for example, a catalyst such as a tertiary amine or a quaternary ammonium salt can be used for accelerating the reaction and, for example, an end-capping agent such as phenol or an alkyl-substituted phenol (e.g., *p*-*tert*-butylphenol) can be used for adjusting the molecular weight.

Usually, the reaction temperature can be set at 0 to 40°C and the reaction time can be set at 1 minute to 5 hours, and the pH during the reaction is preferably maintained at 10 or higher.

A transesterification method (melting method) using a carbonic acid diester as a carbonate precursor can be employed as well. In this method, a prescribed ratio of a dihydric phenol component and a carbonic acid diester are stirred with heating in the presence of an inert gas, and the resulting alcohol or phenol is distilled off.

The reaction temperature varies depending on the boiling point and the like of the resulting alcohol or phenol; however, it is usually in a range of 120 to 350°C. The reaction is carried out while reducing the pressure from the initial stage and thereby distilling off the resulting alcohol or phenol. Further, an ordinary transesterification catalyst can be used for accelerating the reaction.

Examples of the carbonic acid diester used in this transesterification reaction include diphenyl carbonate, dinaphthyl carbonate, dimethyl carbonate, diethyl carbonate, and dibutyl carbonate, among which diphenyl carbonate is particularly preferred.

<Additive (B)>

The polycarbonate resin composition of the present disclosure contains a prescribed amount of an additive (B).

This additive (B) is an additive that can be incorporated into a polycarbonate, and may be composed of plural additives. Preferably, the additive (B) means the following additives (B1) to (B7) that can be incorporated into a polycarbonate.

A total amount of the additive (B) is 0.1 to 1.5 parts by mass with respect to 100 parts by mass of the polycarbonate resin (A). From the standpoints of impact resistance, heat resistance, dry heat resistance and the like, the total amount of the additive (B) is preferably 0.1 to 1.4 parts by mass, more preferably 0.2 to 1.3 parts by mass, still more preferably 0.3 to 1.2 parts by mass, yet still more preferably 0.4 to 0.9 parts by mass, particularly preferably 0.4 to 0.7 parts by mass.

(Ultraviolet Absorber (B1) Represented by Formula 1)

The composition of the present disclosure contains, as the additive (B), an ultraviolet absorber (B1) having a specific benzotriazole skeleton represented by the following Formula 1. From the standpoint of attaining a sufficient effect of reducing the light transmittance at about 420 nm, as well as impact resistance and the like, the ultraviolet absorber (B1) is contained in an amount of not less than 0.1 parts by mass, preferably 0.1 to 1.4 parts by mass, more preferably 0.1 to 1.3 parts by mass, still more preferably 0.2 to 1.2 parts by mass, yet still more preferably 0.3 to 1.1 parts by mass, further more preferably 0.3 to 1.0 parts by mass, particularly preferably 0.4 to 0.8 parts by mass, most preferably 0.4 to 0.6 parts by mass, with respect to 100 parts by mass of the polycarbonate resin (A).

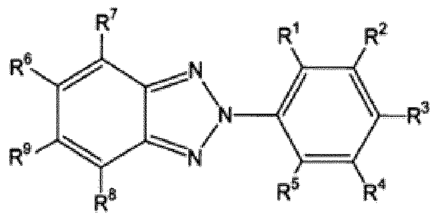

Formula 1

In Formula 1, $R^1$ to $R^5$ each independently represent any of a hydrogen atom, a hydrocarbon group having 1 to 20 carbon atoms, and a hydroxy group, which hydrocarbon group optionally contains an oxygen-containing group. $R^6$ to $R^9$ each independently represent any of a hydrogen atom and a sulfur-containing group represented by R-S-. At least one of $R^6$ to $R^9$ is R-S-, wherein R is a hydrocarbon group having 1 to 24 carbon atoms, or an aromatic group having 6 to 24 carbon atoms in which a hydrogen atom is optionally substituted with an alkyl group having 1 to 18 carbon atoms.

Examples of the hydrocarbon groups include aliphatic hydrocarbon groups, alicyclic hydrocarbon groups, and aromatic hydrocarbon groups.

Examples of the aliphatic hydrocarbon groups include alkyl groups, alkenyl groups, and alkynyl groups. Examples of the alkyl groups include, but not particularly limited to: linear alkyl groups and branched alkyl groups, such as a methyl group, an ethan-1-yl group, a propan-1-yl group, a 1-methylethan-1-yl group, a butan-1-yl group, a butan-2-yl group, a 2-methylpropan-1-yl group, a 2-methylpropan-2-yl group, a pentan-1-yl group, a pentan-2-yl group, a hexan-1-yl group, a heptan-1-yl group, an octan-1-yl group, a 1,1,3,3-tetramethylbutan-1-yl group, a nonan-1-yl group, a decan-1-yl group, an undecan-1-yl group, a dodecan-1-yl group, a tridecan-1-yl group, a tetradecan-1-yl group, a pentadecan-1-yl group, a hexadecan-1-yl group, a heptadecan-1-yl group, an octadecan-1-yl group, a nonadecan-1-yl group, an eicosan-1-yl group, a heneicosan-1-yl group, a docosan-1-yl group, a tricosan-1-yl group, a tetracosan-1-yl group, a benzyl group, and an $\alpha,\alpha$-dimethylbenzyl group. Examples of the alkenyl groups include, but not particularly limited to: linear alkenyl groups and branched alkenyl groups, such as a vinyl group, a prop-1-en-1-yl group, an allyl group, and isopropenyl group, a but-1-en-1-yl group, a but-2-en-1-yl group, a but-3-en-1-yl group, a 2-methylprop-2-en-1-yl group, a 1-methylprop-2-en-1-yl group, a pent-1-en-1-yl group, a pent-2-en-1-yl group, a pent-3-en-1-yl group, a pent-4-en-1-yl group, a 3-methylbut-2-en-1-yl group, a 3-methylbut-3-en-1-yl group, a hex-1-en-1-yl group, a hex-2-en-1-yl group, a hex-3-en-1-yl group, a hex-4-en-1-yl group, a hex-5-en-1-yl group, a 4-methylpent-3-en-1-yl group, a 4-methylpent-3-en-1-yl group, a hept-1-en-1-yl group, a hept-6-en-1-yl group, an oct-1-en-1-yl group, an oct-7-en-1-yl group, a non-1-en-1-yl group, a non-8-en-1-yl group, a dec-1-en-1-yl group, a dec-9-en-1-yl group, an undec-1-en-1-yl group, an undec-10-en-1-yl group, a dodec-1-en-1-yl group, a dodec-11-en-1-yl group, a tridec-1-en-1-yl group, atridec-12-en-1-yl group, a tetradec-1-en-1-yl group, a tetradec-13-en-1-yl group, a pentadec-1-en-1-yl group, a pentadec-14-en-1-yl group, a hexadec-1-en-1-yl group, a hexadec-15-en-1-yl group, a heptadec-1-en-1-yl group, a heptadec-16-en-1-yl group, an octadec-1-en-1-yl group, an octadec-9-en-1-yl group, an octadec-17-en-1-yl group, a nonadec-1-en-1-yl group, an eicos-1-en-1-yl group, a henicos-1-en-1-yl group, a docos-1-en-1-yl group, a tricos-1-en-1-yl group, and a tetracos-1-en-1-yl

group. Examples of the alkynyl groups include, but not particularly limited to: linear alkynyl groups and branched alkynyl groups, such as an ethynyl group, a prop-1-yn-1-yl group, a prop-2-yn-1-yl group, a but-1-yn-1-yl group, a but-3-yn-1-yl group, a 1-methylprop-2-yn-1-yl group, a pent-1-yn-1-yl group, a pent-4-yn-1-yl group, a hex-1-yn-1-yl group, a hex-5-yn-1-yl group, a hept-1-yn-1-yl group, a hept-6-yn-1-yl group, an oct-1-yn-1-yl group, an oct-7-yn-1-yl group, a non-1-yn-1-yl group, a non-8-yn-1-yl group, a dec-1-yn-1-yl group, a dec-9-yn-1-yl group, an undec-1-yn-1-yl group, an undec-10-yn-1-yl group, a dodec-1-yn-1-yl group, a dodec-11-yn-1-yl group, a tridec-1-yn-1-yl group, a tridec-12-yn-1-yl group, a tetradec-1-yn-1-yl group, a tetradec-13-yn-1-yl group, a pentadec-1-yn-1-yl group, a pentadec-14-yn-1-yl group, a hexadec-1-yn-1-yl group, a hexadec-15-yn-1-yl group, a heptadec-1-yn-1-yl group, a heptadec-16-yn-1-yl group, an octadec-1-yn-1-yl group, an octadec-17-yn-1-yl group, a nonadec-1-yn-1-yl group, an eicosa-1-yn-1-yl group, a henicosa-1-yn-1-yl group, a docosa-1-yn-1-yl group, a tricosa-1-yn-1-yl group, and a tetracosa-1-yn-1-yl group.

Examples of the alicyclic hydrocarbon groups include, but not particularly limited to: a cyclopropyl group, a cyclobutyl group, a cyclopentyl group, a cyclohexyl group, a cycloheptyl group, and a cyclooctyl group.

Examples of the aromatic hydrocarbon groups include, but not particularly limited to: groups containing an aromatic ring residue, such as a phenyl residue, a naphthalene residue, or an anthracene residue. Examples of monovalent aromatic hydrocarbon groups include, but not particularly limited to: a phenyl group, a 2-methylphenyl group, a 3-methylphenyl group, a 4-methylphenyl group, a 2,4-dimethylphenyl group, a 2,5-dimethylphenyl group, a 3,4-dimethylphenyl group, a 3,5-dimethylphenyl group, a 2,4,5-trimethylphenyl group, a 2,4,6-trimethylphenyl group, a 4-ethylphenyl group, a 4-propylphenyl group, a 4-isopropylphenyl group, a 4-butylphenyl group, a 4-*tert*-butylphenyl group, a 4-pentylphenyl group, a *4-tert*-pentylphenyl group, a 2,4-bis(4-*tert*-pentyl)phenyl group, a 1,1,3,3-tetramethylbutylphenyl group, a 2-methyl-5-*tert*-butylphenyl group, a 4-pentylphenyl group, a 4-hexylphenyl group, a 4-heptylphenyl group, a 4-octylphenyl group, a 4-nonylphenyl group, a 4-decanylphenyl group, a 4-undecylphenyl group, a 4-dodecylphenyl group, a 4-tridecylphenyl group, a 4-tetradecylphenyl group, a 4-pentadecylphenyl group, a 4-hexadecylphenyl group, a 4-heptadecylphenyl group, a 4-octadecylphenyl group, a 4-biphenyl group, a 2-methoxyphenyl group, a 3-methoxyphenyl group, a 4-methoxyphenyl group, a 2-ethoxyphenyl group, a 3-ethoxyphenyl group, a 4-ethoxyphenyl group, a 2-chlorophenyl group, a 2-fluorophenyl group, a 4-fluorophenyl group, a 2-trifluoromethylphenyl group, a 4-trifluoromethylphenyl group, a 4-hydroxyphenyl group, a 1-naphthyl group, a 2-naphthyl group, a 1-anthracenyl group, a 2-anthracenyl group, and a 9-anthracenyl group. Examples of divalent aromatic hydrocarbon groups include, but not particularly limited to: a 1,4-phenylene group, a 1,3-phenylene group, a 1,2-phenylene group, a 1,8-naphthylene group, a 2,7-naphthylene group, a 2,6-naphthylene group, a 1,4-naphthylene group, a 1,3-naphthylene group, a 9,10-anthracenylene group, a 1,8-anthracenylene group, a 2,7-anthracenylene group, a 2,6-anthracenylene group, a 1,4-anthracenylene group, and a 1,3-anthracenylene group.

Examples of the oxygen-containing group include, but not particularly limited to: a hydroxy group, an alkoxy group, a methoxy group, an ethoxy group, an *n*-propoxy group, an isopropoxy group, an *n*-butoxy group, a sec-butoxy group, an *iso*-butoxy group, a *tert*-butoxy group, a *sec*-pentyloxy group, an *iso*-pentyloxy group, a *tert*-pentyloxy group, a 1-hexyloxy group, a 2-hexyloxy group, a 3-hexyloxy group, a 1-heptyloxy group, a 2-heptyloxy group, a 3-heptyloxy group, a 4-heptyloxy group, a 1-octyloxy group, a 2-octyloxy group, a 3-octyloxy group, a 4-octyloxy group, a 1-nonyloxy group, a 2-nonyloxy group, a 3-nonyloxy group, a 4-nonyloxy group, a 5-nonyloxy group, a 1-decyloxy group, a 2-decyloxy group, a 3-decyloxy group, a 4-decyloxy group, a 5-decyloxy group, a 1-undecyloxy group, a 1-dodecyloxy group, a 1-tridecyloxy group, a 1-tetradecyloxy group, a 1-pentadecyloxy group, a 1-hexadecyloxy group, a 1-heptadecyloxy group, a 1-octadecyloxy group, a phenoxy group, a methylphenoxy group, a dimethylphenoxy group, a naphthoxy group, a phenylmethoxy group, a phenylethoxy group, an acetoxy group, an acetyl group, an aldehyde group, a carboxy group, a urea group, a urethane group, an amide group, an imide group, an ether group, a carbonyl group, an ester group, an oxazole group, a morpholine group, a carbamate group, a carbamoyl group, and a polyoxyethylene group.

Each hydrocarbon group of $R^1$ to $R^5$ in Formula 1 is preferably an alkyl group having 1 to 12 carbon atoms, more preferably an alkyl group having 1 to 4 carbon atoms, particularly preferably a methyl group or a butyl group. Further, at least one of $R^1$ to $R^5$ is preferably a methyl group, and at least one of the remaining $R^1$ to $R^5$ is more preferably a butyl group. It is most preferred that at least one of $R^2$ and $R^4$ be selected from these alkyl groups and $R^1$ be a hydroxy group.

The ultraviolet absorber (B1) has a sulfur-containing group represented by R-S-. Because of the presence of this sulfur-containing group, when the ultraviolet absorber (B1) is incorporated into a polycarbonate resin having a specific viscosity-average molecular weight, the light transmittance at not only 400 nm but also 400 to 420 nm in particular can be reduced while maintaining a high total light transmittance as well as excellent impact resistance, molding residence stability, and dry heat resistance.

In the above-described Formula 1, the sulfur-containing group represented by R-S- is positioned at any one of $R^6$ to $R^9$ and, from the standpoint of making the above-described performance more likely to be expressed, it is preferred that the sulfur-containing group be positioned at $R^6$ or $R^9$.

In the sulfur-containing group, R is a hydrocarbon group having 1 to 24 carbon atoms and, from the standpoint of making the above-described performance more likely to be expressed, R is preferably an alkyl group having 1 to 18

carbon atoms, more preferably an alkyl group having 1 to 12 carbon atoms, particularly preferably alkyl group having 1 to 8 carbon atoms. Further, the alkyl group is preferably linear.

Examples of the aromatic group of R of the sulfur-containing group include, but not particularly limited to, the above-exemplified monovalent aromatic hydrocarbon groups. Thereamong, from the standpoint of making the above-described performance more likely to be expressed, for example, a phenyl residue, a naphthalene residue, a biphenyl residue, and an anthracene residue are preferred, and a phenyl residue is more preferred. The term "residue" used herein is intended to mean a group containing an aromatic moiety and, for example, a "phenyl residue" is intended to mean a benzene ring moiety. Further, a hydrogen atom in the aromatic group of R is optionally substituted with an alkyl group having 1 to 18 carbon atoms and, from the standpoint of making the above-described performance more likely to be expressed, the number of carbon atoms of the alkyl group with which a hydrogen atom of the aromatic group can be substituted is preferably 1 to 12, more preferably 1 to 8, still more preferably 1 to 4.

Surprisingly, it was found that the introduction of the sulfur-containing group represented by R-S- allows an ultraviolet absorber to better inhibit clogging of a vacuum vent pipe system connected to an extruder or a molding machine used for extrusion or molding (this system includes, for example, a vent pipe and a volatile matter trapping device) as compared to a conventional ultraviolet absorber while increasing the molecular weight of the ultraviolet absorber itself. The reason for this is presumed to be because not only the volatility is reduced due to an increase in the molecular weight by that of the sulfur-containing group, but also the ultraviolet absorber (B1) entering as a volatile matter into the vacuum vent pipe system connected to an extruder or a molding machine is less likely to be solidified or crystallized as compared to other conventional ultraviolet absorber, so that a sediment is unlikely to grow to a large volume in the system.

Further, as a result of our intensive studies, it was revealed that, as compared to an ultraviolet absorber in which R of the sulfur-containing group is an aliphatic group, i.e., a sulfur atom-containing substituent does not have an aromatic group, the ultraviolet absorber of the present disclosure in which R of the sulfur-containing group contains an aromatic group can further improve the dry heat resistance of a polycarbonate resin composition.

(Optional Additives)

In the composition of the present disclosure, one or more kinds of the below-exemplified additives other than the ultraviolet absorber (B1) may be incorporated as the additive (B). In this case, from the standpoints of mechanical properties such as impact resistance, as well as heat resistance, dry heat resistance and the like, the additive(s) other than the ultraviolet absorber (B1) can be incorporated, for example, in a range of 0.01 to 0.5 parts by mass, preferably in a range of 0.02 to 0.4 parts by mass, more preferably in a range of 0.03 to 0.3 parts by mass, still more preferably in a range of 0.04 to 0.25 parts by mass, particularly preferably in a range of 0.05 to 0.2 parts by mass, with respect to 100 parts by mass of the polycarbonate resin (A).

a. Phosphorus-Based Heat Stabilizer (B2)

In the composition of the present disclosure, a phosphorus-based heat stabilizer may be incorporated. Examples of the phosphorus-based heat stabilizer include phosphorous acid, phosphoric acid, phosphonous acid, phosphonic acid, and esters thereof. Specific examples thereof include triphenyl phosphite, tris(nonylphenyl)phosphite, tris(2,4-di-tert-butylphenyl)phosphite, tris(2,6-d-*tert*-butylphenyl)phosphite, tridecyl phosphite, trioctyl phosphite, trioctadecyl phosphite, didecyl monophenyl phosphite, dioctyl monophenyl phosphite, diisopropyl monophenyl phosphite, monobutyl diphenyl phosphite, monodecyl diphenyl phosphite, monooctyl diphenyl phosphite, bis(2,6-di-*tert*-butyl-4-methylphenyl)pentaerythritol diphosphite, 2,2-methylene-bis(4,6-di-*tert*-butylphenyl)octyl phosphite, bis(nonylphenyl)pentaerythritol diphosphite, bis(2,4-di-*tert*-butylphenyl)pentaerythritol diphosphite, bis(2,4-di-cumylphenyl)pentaerythritol diphosphite, distearylpentaerythritol diphosphite, tributyl phosphate, triethyl phosphate, trimethyl phosphate, triphenyl phosphate, diphenyl monoorthoxenyl phosphate, dibutyl phosphate, dioctyl phosphate, diisopropyl phosphate, dimethyl benzenephosphonate, diethyl benzenephosphonate, dipropyl benzenephosphonate, tetrakis(2,4-di-*tert*-butylphenyl)-4,4'-biphenylene diphosphonite, tetrakis(2,4-di-*tert*-butylphenyl)-4,3'-biphenylene diphosphonite, tetrakis(2,4-di-*tert*-butylphenyl)-3,3'-biphenylene diphosphonite, bis(2,4-di-tert-butylphenyl)-4-phenyl-phenyl phosphonite, and bis(2,4-di-tert-butylphenyl)-3-phenyl-phenyl phosphonite. These phosphorus-based heat stabilizers may be used singly, or in combination of two or more thereof.

Thereamong, tris(2,4-di-*tert*-butylphenyl)phosphite, tetrakis(2,4-di-*tert*-butylphenyl)-4,4'-biphenylene diphosphonite, bis(2,6-di-*tert*-butyl-4-methylphenyl)pentaerythritol diphosphite, and bis(2,4-di-cumylphenyl)pentaerythritol diphosphite are preferred; bis(2,6-di-*tert*-butyl-4-methylphenyl)pentaerythritol diphosphite and tris(2,4-di-*tert*-butylphenyl)phosphite are more preferred; and tris(2,4-di-*tert*-butylphenyl)phosphite is particularly preferred.

From the standpoints of mechanical properties such as molding residence stability and impact resistance, as well as heat resistance and the like, the content of the phosphorus-based heat stabilizer is preferably 0.01 to 0.1 parts by mass, more preferably 0.01 to 0.05 parts by mass, still more preferably 0.015 to 0.03 parts by mass, with respect to

100 parts by mass of the polycarbonate resin (A).

b. Fatty Acid Ester-Based Mold Release Agent (B3)

In the composition of the present disclosure, a fatty acid ester-based mold release agent may be incorporated so as to, for example, improve the releasability of a molded article from a mold upon melt-molding.

As the fatty acid ester-based mold release agent, for example, at least one mold release agent selected from the group consisting of (i) esters of a monohydric alcohol having 1 to 20 carbon atoms with a saturated or unsaturated fatty acid having 10 to 30 carbon atoms and (ii) partial and full esters of a polyhydric alcohol having 1 to 25 carbon atoms with a saturated or unsaturated fatty acid having 10 to 30 carbon atoms.

Examples of the (i) esters of a monohydric alcohol having 1 to 20 carbon atoms with a saturated or unsaturated fatty acid having 10 to 30 carbon atoms include stearyl stearate, palmityl palmitate, butyl stearate, methyl laurate, and isopropyl palmitate, among which stearyl stearate is preferred.

Examples of the (ii) partial and full esters of a polyhydric alcohol having 1 to 25 carbon atoms with a saturated or unsaturated fatty acid having 10 to 30 carbon atoms include glycerol monostearate, glycerol distearate, glycerol tristearate, glycerol monobehenate, glycerol tribehenate, glycerol trisorbate, pentaerythritol monostearate, pentaerythritol tetrastearate, pentaerythritol tetrapelargonate, propylene glycol monostearate, sorbitan monostearate, 2-ethylhexy stearate, and full esters and partial esters of dipentaerythritol, such as dipentaerythritol hexastearate.

Among these fatty acid esters, a mixture of pentaerythritol tetrastearate, glycerol tristearate or glycerol tristearate with stearyl stearate is preferably used, and a mixture of glycerol tristearate with stearyl stearate is particularly preferably used. The mixing weight ratio (former/latter) is preferably 45 to 15/55 to 85, more preferably 40 to 15/60 to 85.

From the standpoints of impact resistance, heat resistance, mold releasability, discoloration resistance and the like, the content of the fatty acid ester-based mold release agent is preferably 0.03 to 0.5 parts by mass, more preferably 0.08 to 0.4 parts by mass, still more preferably 0.1 to 0.3 parts by mass, with respect to 100 parts by mass of the polycarbonate resin (A).

The fatty acid ester-based mold release agent can be used in combination with other mold release agent known to those of ordinary skill in the art and, in such a case, the content of the fatty acid ester-based mold release agent is preferably 0.02 to 0.45 parts by mass, and it is preferred that the fatty acid ester-based mold release agent be a main component (50% or higher) of mold release agents.

From the standpoints of molding residence stability and the like at a high temperature, the lower the content of trace metal impurities, particularly tin (Sn) in the fatty acid ester-based mold release agent, the more preferred it is, and the content of trace metal impurities is preferably 200 ppm or less, more preferably 100 ppm or less, still more preferably 50 ppm or less, particularly preferably 1 ppm or less. The amount of each metal in a mold release agent can be analyzed by ICP (inductively-coupled plasma) spectroscopy.

c. Hindered Phenol-Based Antioxidant (B4)

In the composition of the present disclosure, a hindered phenol-based antioxidant may be incorporated. By incorporating such an antioxidant, for example, an effect of inhibiting hue deterioration in molding and discoloration of the resulting molded article in the long-term use under a high temperature (dry heat resistance) can be exerted.

Examples of the hindered phenol-based stabilizer include *n*-octadecyl-*β*-(4'-hydroxy-3',5'-di-*tert*-butylphenyl)propionate, 2,6-di-*tert*-butyl-4-(*N*,*N*-dimethylaminomethyl)phenol, 3,5-di-*tert*-butyl-4-hydroxybenzylphosphonate diethyl ester, 2,2'-methylene-bis(4-methyl-6-*tert*-butylphenol), 2,2'-methylene-bis(4-ethyl-6-*tert*-butylphenol), 4,4'-methylene-bis(2,6-di-*tert*-butylphenol), 2,2'-methylene-bis(4-methyl-6-cyclohexylphenol), 1,6-hexanediol-bis[3-(3,5-di-*tert*-butyl-4-hydroxyphenyl)propionate], bis[2-*tert*-butyl-4-methyl-6-(3-*tert*-butyl-5-methyl-2-hydroxybenzyl)phenyl]terephthalate, 4,4'-thiobis(3-methyl-6-*tert*-butylphenol), 2,2'-thiobis(4-methyl-6-*tert*-butylphenol), bis(3,5-di-*tert*-butyl-4-hydroxybenzyl)sulfide, 4,4'-di-thiobis(2,6-di-*tert*-butylphenol), 4,4'-tri-thiobis(2,6-di-*tert*-butylphenol), 2,2-thiodiethylene-bis[3-(3,5-di-*tert*-butyl-4-hydroxyphenyl)propionate], and tetrakis[methylene-3-(3',5'-di-*tert*-butyl-4-hydroxyphenyl)propionate]methane. These are all available as commercial products. The above-exemplified hindered phenol-based stabilizers may be used singly, or in combination of two or more thereof.

From the standpoints of impact resistance, dry heat resistance and the like, the content of the hindered phenol-based antioxidant is preferably 0.01 to 0.30 parts by mass, more preferably 0.02 to 0.25 parts by mass, still more preferably 0.03 to 0.15 parts by mass, with respect to 100 parts by mass of the polycarbonate resin (A).

d. Epoxy Group-Containing Compound (B5)

In the composition of the present disclosure, an epoxy group-containing compound may be incorporated. The epoxy group-containing compound is incorporated for the purposes of inhibiting mold corrosion and improving the moist

heat resistance and, basically, compounds having an epoxy functional group are all applicable.

Specific examples of a preferred epoxy group-containing compound include 3,4-epoxycyclohexylmethyl-3',4'-epoxycyclohexyl carboxylate, 1,2-epoxy-4-(2-oxiranyl)cyclohexane adduct of 2,2-bis(hydroxymethyl)-1-butanol, a copolymer of methyl methacrylate and glycidyl methacrylate, and a copolymer of styrene and glycidyl methacrylate.

From the standpoints of impact resistance, heat resistance, molding residence stability, transparency, mold corrosion-inhibiting effect, moist heat resistance-improving effect and the like, the content of the epoxy group-containing compound is preferably 0.001 to 0.02 parts by mass, more preferably 0.004 to 0.15 parts by mass, still more preferably 0.005 to 0.1 parts by mass, with respect to 100 parts by mass of the polycarbonate resin (A).

e. Ultraviolet Absorber (B6) Other Than Ultraviolet Absorber (B1)

In the composition of the present disclosure, in addition to the ultraviolet absorber (B1), for example, a benzotriazole-based ultraviolet absorber, a benzophenone-based ultraviolet absorber, a triazine-based ultraviolet absorber, a cyclic imino ester-based ultraviolet absorber, or a cyanoacrylate-based ultraviolet absorber may be incorporated.

Examples thereof include 2-(2'-hydroxy-5'-methylphenyl)benzotriazole, 2-(2'-hydroxy-5'-*tert*-octylphenyl)benzotriazole, 2-(2'-hydroxy-3',5'-dicumylphenyl)phenylbenzotriazole, 2-(2'-hydroxy-3'-*tert*-butyl-5'-methylphenyl)-5-chlorobenzotriazole, 2,2'-methylene-bis[4-(1,1,3,3-tetramethylbutyl)-6-(2*N*-benzotriazol-2-yl)phenol], 2-(2'-hydroxy-3',5'-di-tert-butylphenyl)benzotriazole, 2-(2'-hydroxy-3',5'-di-*tert*-butylphenyl)-5-chlorobenzotriazole, 2-(2'-hydroxy-3',5'-di-*tert*-amylphenyl)benzotriazole, 2-(2'-hydroxy-5'-tert-octylphenyl)benzotriazole, 2-(2'-hydroxy-5'-*tert*-butylphenyl)benzotriazole, 2-(2'-hydroxy-4'-octoxyphenyl)benzotriazole, 2,2'-methylene-bis(4-cumyl-6-benzotriazolephenyl), 2,2'-p-phenylene-bis(1,3-benzoxazin-4-one), and 2-[2-hydroxy-3-(3,4,5,6-tetrahydrophthalimidemethyl)-5-methylphenyl]benzotriazole. These ultraviolet absorbers may be used singly, or as a mixture of two or more thereof.

Thereamong, 2-(2'-hydroxy-5'-methylphenyl)benzotriazole, 2-(2'-hydroxy-5'-*tert*-octylphenyl)benzotriazole, 2-(2'-hydroxy-3',5'-dicumylphenyl)phenylbenzotriazole, 2-(2'-hydroxy-3'-*tert*-butyl-5'-methylphenyl)-5-chlorobenzotriazole, 2,2'-methylene-bis[4-(1,1,3,3-tetramethylbutyl)-6-(2H-benzotriazol-2-yl)phenol], and 2-[2-hydroxy-3-(3,4,5,6-tetrahydrophthalimidemethyl)-5-methylphenyl]benzotriazole are preferred; and 2-(2'-hydroxy-5'-*tert*-octylphenyl)benzotriazole and 2,2'-methylene-bis[4-(1,1,3,3-tetramethylbutyl)-6-(2H-benzotriazol-2-yl)phenol] are more preferred.

In the composition of the present disclosure, from the standpoints of impact resistance, heat resistance, transparency, moldability and hue, the content of an ultraviolet absorber (particularly a benzotriazole-based ultraviolet absorber) other than the ultraviolet absorber (B1) is preferably 0.05 to 0.3 parts by mass, more preferably 0.1 to 0.25 parts by mass, still more preferably 0.15 to 0.25 parts by mass, with respect to 100 parts by mass of the polycarbonate resin (A).

f. Bluing Agent (B7)

In the composition of the present disclosure, a bluing agent may be incorporated for the purpose of offsetting a yellow tinge of a molded article caused by the polycarbonate resin and ultraviolet absorber. The bluing agent is not particularly restricted as long as it can be used in a polycarbonate resin. An anthraquinone-based dye is generally preferred because of its availability.

Representative examples of a specific bluing agent include Solvent Violet 13 (generic name) [CA. No. (color index No) 60725; trademarks "MACROLEX VIOLET B" manufactured by Bayer AG, "DIA RESIN BLUE G" manufactured by Mitsubishi Chemical Corporation, and "SUMIPLAST VIOLET B" manufactured by Sumitomo Chemical Co., Ltd.], Solvent Violet 31 (generic name) [CA. No. 68210; trademark "DIA RESIN VIOLET D" manufactured by Mitsubishi Chemical Corporation], Solvent Violet 33 (generic name) [CA. No 60725; trademark "DIA RESIN BLUE J" manufactured by Mitsubishi Chemical Corporation], Solvent Blue 94 (generic name) [CA. No. 61500; trademark "DIA RESIN BLUE N" manufactured by Mitsubishi Chemical Corporation], Solvent Violet 36 (generic name) [CA. No. 68210; trademark "MACROLEX VIOLET 3R" manufactured by Bayer AG], Solvent Blue 97 (generic name) [trademark "MACROLEX BLUE RR" manufactured by Bayer AG], and Solvent Blue 45 (generic name) [CA. No. 61110; trademark "POLYSYNTHLEN BLUE RLS") manufactured by Clariant Japan K.K.].

Taking into consideration the visual transmittance and the like, the bluing agent can be usually contained in the polycarbonate resin at a concentration of 0.3 to 1.2 ppm.

In the composition of the present disclosure, for example, a heat stabilizer other than the above-described ones, an antistatic agent, a flame retardant, a heat ray shielding agent, a fluorescent brightener, a pigment, a light diffusing agent, a reinforcing filler, other resin, and an elastomer may further be incorporated within a range that does not impair the object of the present disclosure.

The other heat stabilizer is, for example, a sulfur-based heat stabilizer. Examples of the sulfur-based heat stabilizer include pentaerythritol-tetrakis(3-laurylthiopropionate), pentaerythritol-tetrakis(3-myristylthiopropionate), pentaerythritol-tetrakis(3-stearylthiopropionate), dilauryl-3,3'-thiodipropionate, dimyristyl-3,3'-thiodipropionate, and distearyl-3,3'-thiodipropionate. Thereamong, pentaerythritol-tetrakis(3-laurylthiopropionate), pentaerythritol-tetrakis(3-myristylthiopropionate), dilauryl-3,3'-thiodipropionate, and dimyristyl-3,3'-thiodipropionate are preferred, and pentaerythritol-tetrakis(3-laurylthiopropionate) is particularly preferred. These thioether compounds are commercially available from Sumitomo Chemical Co., Ltd. as SUMILIZER TP-D (trade name), SUMILIZER TPM (trade name) and the like, and can be easily obtained.

In the composition of the present disclosure, the content of the sulfur-based heat stabilizer is preferably 0.001 to 0.2 parts by mass with respect to 100 parts by mass of the polycarbonate resin (A).

<Hot-Melt-Molded Article>

The term "hot-melt-molded article" used herein is intended to mean a transparent molded body obtained by hot-melt molding of the polycarbonate resin composition of the present disclosure in accordance with a molding method which involves heating and melting, such as injection molding, extrusion molding, or rotational molding. Specific examples of the hot-melt-molded article include: planar molded articles (e.g., injection-molded flat plates and extrusion-molded flat plates (such as extrusion-molded sheets)) that are used as front panels of various displays of personal computers, car navigation systems and the like, covers and front panels of various light emitters, and covers and front panels of various light sources; planar molded articles that are used as window glasses (e.g., injection-molded sheets and extrusion-molded sheets); and optical lenses such as spectacle lenses. The surfaces of these molded articles may be shaped, and the planar molded articles may be further press-molded and thereby imparted with a curved shape or a three-dimensional shape. These molded articles all have excellent impact resistance, maintain a high light transmittance, and exhibit an effect of reducing the light transmittance at not only 400 nm, but also 400 to 420 nm in particular.

The thickness of a molded article is not particularly restricted; however, from the standpoint of the practical strength of the molded article, the thickness is preferably 0.3 mm or greater, more preferably 0.5 mm or greater, still more preferably 1.0 mm or greater, particularly preferably 2 mm or greater. An upper limit value of the thickness is not particularly restricted; however, from the standpoint of moldability, it is preferably 10 mm or less, more preferably 7 mm or less, still more preferably 5 mm or less.

<Spectacle Lens>

Examples of a spectacle lens, which is one kind of hot-melt-molded article, include a finished lens whose convex and concave surfaces are both optically finished by transfer of glass mold surfaces thereto at the time of molding and which is molded in accordance with the desired lens power. Examples also include a semi-finished lens on which only the concave surface is optically finished in the same manner as in a finished lens and the concave side will be later optically finished in accordance with the desired lens power upon an order.

The semi-finished lens is ground or cut using a curve generator, an NC-controlled cutting machine or the like depending on a necessary concave surface processing, and a smoothing treatment (fining) is performed thereon as required. The thus cut or ground and smoothened (fined) surface is polished and mirror-finished using, for example, a polishing dish with an intervening abrasive agent or abrasive cloth, or a flexible polishing tool, whereby the surface is optically finished.

Thereafter, the finished lens and the thus polished semi-finished lens are both washed and examined for damages, foreign matters and the like. Further, after optionally performing, for example, the dying step of dying the lens in a desired color, the hard-coating step of forming a cured film for covering the plastic lens that is easily scratched, and the film-forming step of forming an anti-reflection film for reducing the surface reflection and improving the transmittance of the lens, the resulting finished lenses are shipped as complete products to be used by users.

Spectacle lenses formed from the polycarbonate resin composition of the present disclosure can be used as vision corrective lenses, sunglass lenses, lenses of protective glasses, and the like. These spectacle lenses all maintain a high light transmittance and have an effect of reducing the transmittance of light of about 420 nm, particularly light of 400 nm to 420 nm.

EXAMPLES

The present disclosure will now be described more concretely by way of Examples thereof; however, the present disclosure is not restricted to the below-described Examples.

<<Examples 1 to 24 and Comparative Examples 1 to 16>>

The following evaluations were performed for polycarbonate resin compositions obtained by the below-described method, and the results thereof are shown in Tables 1 to 3. In Tables 1 to 3, the numerical values of polycarbonate resins and additives are all in parts by mass.

<Charpy Impact Strength (Impact Resistance)>

A pellet obtained in each Example was dried in a hot air dryer at 120°C for 5 hours and molded into a test piece of 10 mm in width, 80 mm in length and 4 mm in thickness using a J-75EIII injection molding machine manufactured by The Japan Steel Works, Ltd. at a cylinder temperature of 280°C and a mold temperature of 70 to 75°C in a 1-minute cycle, and the notched Charpy impact strength was measured in accordance with ISO179.

<Heat Deflection Temperature under Load (Heat Resistance)>

A pellet obtained in each Example was dried in a hot air dryer at 120°C for 5 hours and molded into a test piece of 10 mm in width, 80 mm in length and 4 mm in thickness using a J-75EIII injection molding machine manufactured by The Japan Steel Works, Ltd. at a cylinder temperature of 280°C and a mold temperature of 70 to 75°C in a 1-minute cycle, and the heat deflection temperature was measured under a load of 1.80 MPa in accordance ISO75-1 and 75-2.

<Total Light Transmittance>

A pellet obtained in each Example was dried in a hot air dryer at 120°C for 5 hours and, using a J-75EIII injection molding machine manufactured by The Japan Steel Works, Ltd. at a cylinder temperature of 350°C and a mold temperature of 80°C in a 1-minute cycle, the thus dried pellet was molded into a three-step plate having a width of 50 mm, a length of 90 mm, and a thickness of 3 mm (20 mm in length), 2 mm (45 mm in length) and 1 mm (25 mm in length) from the gate side to obtain a test piece. The total light transmittance in the 2 mm-thick part of this three-step plate was measured in accordance with JIS K7361 using NDH-2000 manufactured by Nippon Denshoku Industries Co., Ltd.

<Spectral Transmittance>

A pellet obtained in each Example was molded using an injection molding machine (cylinder temperature: 350°C, 1-minute cycle) to obtain a measurement flat plate (90 mm in length × 50 mm in width × 2 mm in thickness). The spectral transmittance of this measurement flat plate in a wavelength range of 300 nm to 500 nm was measured using Cary 5000 manufactured by Varian Inc. to determine the spectral transmittance at 420 nm and 400 nm.

<Molding Residence Test>

A molding residence test corresponding to a molding heat resistance test was conducted to evaluate the molding residence stability. A pellet obtained in each Example was dried in a hot air dryer at 120°C for 5 hours and, using an injection molding machine J85-ELIII manufactured by The Japan Steel Works, Ltd. at a cylinder temperature of 350°C and a mold temperature of 80°C in a 1-minute cycle, the thus dried pellet was molded into a three-step plate having a width of 50 mm, a length of 90 mm, and a thickness of 3 mm (20 mm in length), 2 mm (45 mm in length) and 1 mm (25 mm in length) from the gate side. After continuously molding the three-step plate for 20 shots, a resin was retained for 10 minutes in the cylinder of the injection molding machine at a cylinder temperature of 350°C. The hue (L, a, b) of the 2 mm-thick part of this test piece was measured before and after the retention by an illuminant-C transmission method using Color-Eye 7000A manufactured by Gretag Macbeth LLC in accordance with JIS K7105, and the color difference ΔE' was calculated using the following Equation 4. A smaller ΔE' means a higher molding residence stability.

$$\Delta E' = \{(\Delta L')^2 + (\Delta a')^2 + (\Delta b')^2\}^{1/2} \qquad \text{Equation 4}$$

Hue of molded plate before retention: L", a", b"
Hue of molded plate after retention: L"", a"", b""

$$\Delta L': L'' - L''''$$

$$\Delta a': a'' - a''''$$

$$\Delta b': b'' - b''''$$

<Dry Heat Resistance Test>

A pellet obtained in each Example was dried in a hot air dryer at 120°C for 5 hours and, using an injection molding machine J85-ELIII manufactured by The Japan Steel Works, Ltd. at a cylinder temperature of 350°C and a mold temperature of 80°C in a 1-minute cycle, the thus dried pellet was molded into a three-step plate having a width of 50 mm, a length of 90 mm, and a thickness of 3 mm (20 mm in length), 2 mm (45 mm in length) and 1 mm (25 mm in length) from the gate side to obtain a test piece.

A dry heat resistance test was conducted by treating the thus obtained test piece in a hot air dryer at 130°C for 500 hours. The hue of the 2 mm-thick part of this test piece was measured before and after the treatment by an illuminant-C transmission method using Color-Eye 7000A manufactured by Gretag Macbeth LLC, and the yellowing degree before and after the test (ΔYI) was calculated based on the following Equation 5. A smaller ΔYI means a smaller hue change and a higher dry heat resistance.

$$\Delta YI = YI - YI_0 \qquad \text{Equation 5}$$

ΔYI: yellowing degree before and after test
YI: yellowness after test
$YI_0$: yellowness before test

<Synthesis of Ultraviolet Absorber (B1)>

As an ultraviolet absorber (B1), a compound represented by the following Formula 5, which was obtained in the following Synthesis Example 1, was used.

(Synthesis Example 1)

In 50 mL of DMF, 2-(2'-hydroxy-3'-*tert*-butyl-5'-methylphenyl)-5-chlorobenzotriazole (5.00 g, 15.8 mmol), octanethiol (7.63 g, 52.1 mmol), potassium carbonate (7.20 g, 52.1 mmol), and potassium iodide (0.18 g, 1.1 mmol) were allowed to react at 150°C for 20 hours. After the completion of the reaction, the resultant was purified to obtain a compound represented by the following Formula 5 (UV-1). The analysis results of this compound are shown below.

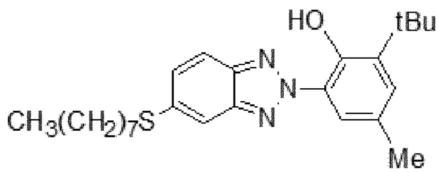

Formula 5

Analysis by FT-IR (KBr)

3,125 $cm^{-1}$: O-H stretching vibration
1,438, 1,391 $cm^{-1}$: triazole ring stretching vibration
661 $cm^{-1}$: C-S stretching vibration

Analysis by $^1H$-NMR ($CDCl_3$, 400 MHz)
δ 0.88(t, 3H, $CH_3(CH_2)_7$-S), 1.27(m, 8H, $CH_3(CH_2)_4(CH_2)_3$-S), 1.49(m, 11H, -Ph-OH-$CH_3$-$C(CH_3)_3$, $CH_3(CH_2)_4CH_2(CH_2)_2$-S), 1.75(quin, 2H, $CH_3(CH_2)_5CH_2CH_2$-S), 2.38(s, 3H,-Ph-OH-$CH_3$-$C(CH_3)_3$), 3.03(t, 2H, $CH_3(CH_2)_5CH_2CH_2$-S), 7.16(s, 1H), 7.37(d, 1H), 7.70(s, 1H), 7.81(d, 1H), 8.05(s, 1H), (insg.5$_{arom}$.CH), 11.61(s, 1H, -Ph-OH-$CH_3$-$C(CH_3)_3$)

Analysis by $^{13}C$-NMR ($CDCl_3$, 400 MHz)
δ 14.0($\underline{C}H_3(CH_2)_7$-S), 20.0(-Ph-OH-$\underline{C}H_3$-$C(CH_3)_3$), 22.6(-Ph-OH-$CH_3$-$\underline{C}(CH_3)_3$), 28.7($CH_3(\underline{C}H_2)_5CH_2CH_2$-S), 31.9(-Ph-OH-$CH_3$-$C(\underline{C}H_3)_3$), 33.2($CH_3(CH_2)_5\underline{C}H_2CH_2$-S), 35.4($CH_3(CH_2)_5CH_2\underline{C}H_2$-S), 113.6, 117.5, 119.3, 128.7, 129.3($\underline{C}H_{arom}$), 141.2, 143.4($\underline{C}_{arom}$), 125.4($\underline{C}_{arom}$-N), 128.3($C_{arom}$-$\underline{C}H_3$), 138.0($\underline{C}_{arom}$-S), 139.1($\underline{C}_{arom}$-$C(CH_3)_3$), 146.7($\underline{C}_{arom}$-OH)

(Synthesis Example 2)

In 62.5 g of DMF, 2-(2-hydroxy-3-*tert*-butyl-5-methylphenyl)-5-chlorobenzotriazole (25.0 g, 79.2 mmol), benzenethiol (17.4 g, 158.3 mmol), potassium carbonate (24.1 g, 174.2 mmol), and potassium iodide (0.9 g, 5.5 mmol) were allowed to at 125°C for 12 hours. After the completion of the reaction, the resultant was purified to obtain a compound represented by the following Formula 6 (UV-4). The analysis results of this compound are shown below.

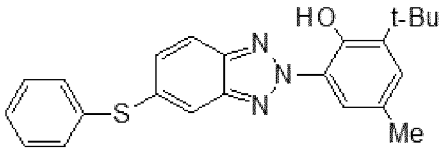

Formula 6

Analysis by FT-IR (KBr)

3,000 $cm^{-1}$: O-H stretching vibration
1,445, 1,390 $cm^{-1}$: triazole ring stretching vibration
665 $cm^{-1}$: C-S stretching vibration

Analysis by $^{1}H$-NMR ($CDCl_3$, 400 MHz)
δ 1.48(s, 9H, -Ph-OH-$CH_3$-C($CH_3$)$_3$), 2.37(s, 3H, -Ph-OH-$CH_3$-C($CH_3$)$_3$), 7.16(s, 1H), 7.38(d, 4H), 7.48(s, 2H), 7.68(s, 1H), 7.83(d, 1H), 8.03(d, 1H), (insg.10arom.CH), 11.55(s, 1H,-Ph-OH-$CH_3$-C($CH_3$)$_3$)
Analysis by $^{13}C$-NMR ($CDCl_3$, 400 MHz)
δ 20.9(-Ph-OH-$\underline{C}H_3$-C($CH_3$)$_3$), 29.5(-Ph-OH-$CH_3$-C($CH_3$)$_3$), 35.4(-Ph-OH-$CH_3$-$\underline{C}$($CH_3$)$_3$), 116.8, 118.0, 119.3, 128.3, 128.8, 129.6, 132.7($CH_{arom}$), 125.5, 141.2, 143.2($C_{arom}$), 129.8($C_{arom}$-$CH_3$), 139.2($\underline{C}_{arom}$-S), 139.2(S-$\underline{C}_{arom}$), 139.2($\underline{C}_{arom}$-C($CH_3$)$_3$), 146.7($\underline{C}_{arom}$-OH)

(Synthesis Example 3)

In 62.5 g of DMF, 2-(2'-hydroxy-3'-*tert*-butyl-5'-methylphenyl)-5-chlorobenzotriazole (25.0 g, 79.2 mmol), 4-isopropyl benzenethiol (24.1 g, 158.3 mmol), potassium carbonate (24.1 g, 174.2 mmol), and potassium iodide (0.92 g, 5.54 mmol) were allowed to react at 125°C for 12 hours. After the completion of the reaction, the resultant was purified to obtain a compound represented by the following Formula 7 (UV-5). The analysis results of this compound are shown below.

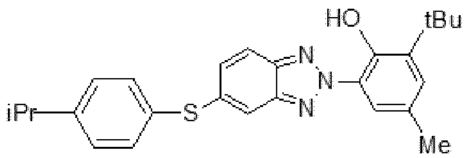

Formula 7

Analysis by FT-IR (KBr)

3,000 $cm^{-1}$: O-H stretching vibration
1,446, 1,389 $cm^{-1}$: triazole ring stretching vibration
666 $cm^{-1}$: C-S stretching vibration

Analysis by $^{1}H$-NMR ($CDCl_3$, 400 MHz)
δ 1.30(d, 6H, ($CH_3$)$_2$CH-Ph-S-), 1.48(s, 9H, -Ph-OH-$CH_3$-C($CH_3$)$_3$), 2.37(s, 3H, -Ph-OH-$CH_3$-C($CH_3$)$_3$), 2.95(m, 1H, ($CH_3$)$_2$CH-Ph-S-), 7.16(s, 1H), 7.28(s, 2H), 7.36(d, 1H), 7.45(s, 2H), 7.57(s, 1H), 7.81(d, 1H), 8.02(d, 1H), (insg.$9_{arom}$.CH), 11.58(s, 1H, -Ph-OH-$CH_3$-C($CH_3$)$_3$)
Analysis by $^{13}C$-NMR ($CDCl_3$, 400 MHz)

δ 20.9(-Ph-OH-$\underline{CH}_3$-C($CH_3$)$_3$), 23.9(($\underline{CH}_3$ )$_2$ CH-Ph-S-), 29.5(-Ph-OH-$CH_3$-C($\underline{CH}_3$)$_3$), 33.9(($CH_3$)$_2$$\underline{CH}$-Ph-S-), 35.4(-Ph-OH-$CH_3$-$\underline{C}$($CH_3$)$_3$), 115.3, 117.8, 119.3, 127.9, 128.7, 129.2, 129.6, 133.6($CH_{arom}$), 125.4, 141.4, 143.3($C_{arom}$), 128.3($C_{arom}$-$\underline{CH}_3$), 138.5($\underline{C}_{arom}$-S), 138.5(S-$\underline{C}_{arom}$), 139.1($\underline{C}_{arom}$-C($CH_3$)$_3$), 146.7($\underline{C}_{arom}$-OH), 149.7($C_{arom}$-$\underline{CH}$)

(Synthesis Example 4)

In 50 mL of DMF, 2-(2'-hydroxy-3'-*tert*-butyl-5'-methylphenyl)-5-chlorobenzotriazole (5.00 g, 15.8 mmol), octanethiol (7.63 g, 52.1 mmol), potassium carbonate (7.20 g, 52.1 mmol), and potassium iodide (0.18 g, 1.1 mmol) were allowed to react at 150°C for 20 hours. After the completion of the reaction, the resultant was purified to obtain a compound represented by the following Formula 8 (UV-6). This compounds corresponds to an ultraviolet absorber (B6) other than the ultraviolet absorber (B1) of the present disclosure. The analysis results of this compound are shown below.

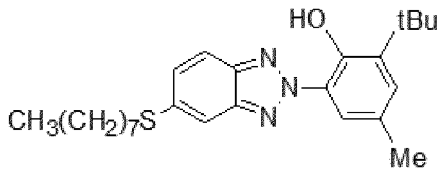

Formula 8

Analysis by FT-IR (KBr)

3,125 $cm^{-1}$: O-H stretching vibration
1,438, 1,391 $cm^{-1}$: triazole ring stretching vibration
661 $cm^{-1}$: C-S stretching vibration

Analysis by $^1H$-NMR ($CDCl_3$, 400 MHz)
δ 0.88(t, 3H, $CH_3(CH_2)_7$-S), 1.27(m, 8H, $CH_3(CH_2)_4(CH_2)_3$-S), 1.49(m, 11H, -Ph-OH-$CH_3$-$C(CH_3)_3$, $CH_3(CH_2)_4CH_2(CH_2)_2$-S), 1.75(quin, 2H, $CH_3(CH_2)_5CH_2CH_2$-S), 2.38(s, 3H,-Ph-OH-$CH_3$-$C(CH_3)_3$), 3.03(t, 2H, $CH_3(CH_2)_SCH_2CH_2$-S), 7.16(s, 1H), 7.37(d, 1H), 7.70(s, 1H), 7.81(d, 1H), 8.05(s, 1H), (insg.5arom.CH), 11.61(s, 1H, -Ph-OH-$CH_3$-$C(CH_3)_3$)
Analysis by $^{13}C$-NMR ($CDCl_3$, 400 MHz)
δ 14.0($\underline{CH}_3(CH_2)_7$-S), 20.0(-Ph-OH-$\underline{CH}_3$-$C(CH_3)_3$), 22.6(-Ph-OH-$CH_3$-$\underline{C}(CH_3)_3$), 28.7($CH_3(\underline{CH}_2)_5CH_2CH_2$-S), 31.9(-Ph-OH-$CH_3$-$C(\underline{CH}_3)_3$), 33.2($CH_3(CH_2)_5\underline{CH}_2CH_2$-S), 35.4($CH_3(CH_2)_5CH_2CH_2$-S), 113.6, 117.5, 119.3, 128.7, 129.3($\underline{CH}_{arom}$), 141.2, 143.4($\underline{C}_{arom}$), 125.4($\underline{C}_{arom}$-N), 128.3($C_{arom}$-$\underline{CH}_3$), 138.0($\underline{C}_{arom}$-S), 139.1($\underline{C}_{arom}$-$C(CH_3)_3$), 146.7($\underline{C}_{arom}$-OH)

(Example 1)

To 100 parts of a polycarbonate resin granule having a viscosity-average molecular weight of 23,900 (PC-1) which was obtained by polymerizing bisphenol A with phosgene by a conventional interfacial polymerization method, 0.22 parts of the compound obtained in Synthesis Example 1 (UV-1) as an ultraviolet absorber, 0.03 parts of a phosphorus-based heat stabilizer (P-1), 0.1 parts of a fatty acid ester-based mold release agent (R-1) as a mold release agent, and 0.00025 parts of a bluing agent (BL-1) were added, and the resultant was thoroughly mixed using a tumbler and subsequently pelletized using a 30-mmφ vent-type twin-screw extrusion molding machine at a temperature of 290°C and a vacuum degree of 4.7 kPa.

(Examples 2 to 24 and Comparative Examples 1 to 16)

The same operations as in Example 1 were performed, except that the materials were used in the respective amounts shown in Tables 1 to 3. The evaluation results thereof are shown in Tables 1 to 3. The components corresponding to the respective symbols used in Tables 1 and 3 are shown below.

(Polycarbonate Resin Granules)

PC-1: an aromatic polycarbonate resin powder having a viscosity-average molecular weight of 23,900, which was obtained by polymerization of bisphenol A and phosgene by an interfacial polymerization method (manufactured by Teijin Chemicals Ltd.: PANLITE (trademark) L-1250WP)
PC-2: an aromatic polycarbonate resin powder having a viscosity-average molecular weight of 22,200, which was obtained by polymerization of bisphenol A and phosgene by an interfacial polymerization method (manufactured by Teijin Chemicals Ltd.: PANLITE (trademark) L-1225WP)

PC-3: an aromatic polycarbonate resin powder having a viscosity-average molecular weight of 19,700, which was obtained by polymerization of bisphenol A and phosgene by an interfacial polymerization method (manufactured by Teijin Chemicals Ltd.: PANLITE (trademark) L-1225WX).

(Ultraviolet Absorbers)

UV-1: 2-(2'-hydroxy-3'-*tert*-butyl-5'-methylphenyl)-5-octylsulfanylbenzotriazole (compound obtained in Synthesis Example 1)
UV-2: 2-(2'-hydroxy-5'-*tert*-octylphenyl)benzotriazole (manufactured by BASF Ltd.: TINUVIN (trademark) 329)
UV-3: 2-(2'-hydroxy-3'-*tert*-butyl-5'-methylphenyl)-5-chlorobenzotriazole (manufactured by BASF Ltd.: TINUVIN (trademark) 326)
UV-4: 2-(2'-hydroxy-3'-*tert*-butyl-5'-methylphenyl)-5-phenylsulfanylbenzotriazole (compound obtained in Synthesis Example 2)
UV-5: 2-(2'-hydroxy-3'-*tert*-butyl-5'-methylphenyl)-5-(4'-isopropylphenyl)sulfanylbenzotriazole (compound obtained in Synthesis Example 3)
UV-6: 2-(2'-hydroxy-3'-*tert*-butyl-5'-methylphenyl)-5-octylsulfanylbenzotriazole (compound obtained in Synthesis Example 4)

(Phosphorus-Based Heat Stabilizers)

P-1: tris(2,4-di-*tert*-butylphenyl)phosphite (manufactured by BASF Ltd.: IRGAFOS (trademark) 168)
P-2: bis(2,6-di-*tert*-butyl-4-methylphenyl)pentaerythritol diphosphite (manufactured by ADEKA Corporation: ADK STAB (trademark) PEP-36)

(Fatty Acid Ester-Based Mold Release Agents)

R-1: a mixture of stearic acid triglyceride and stearyl stearate (manufactured by Riken Vitamin Co., Ltd.: RIKEMAL (trademark) SL-900)
R-2: pentaerythritol tetrastearate (manufactured by Emery Oleochemicals (M) Sdn Bhd: LOXIOL (trademark) VPG861)

(Antioxidants)

H-1: a hindered phenol-based antioxidant; octadecyl-3-(3,5-di-*tert*-butyl-4-hydroxyphenyl)propionate (manufactured by BASF Ltd.: IRGANOX (trademark) 1076)
H-2: a hindered phenol-based antioxidant; pentaerythritol-tetrakis(3-(3,5-di-*tert*-butyl-4-hydroxyphenyl)propionate) (manufactured by BASF Ltd.: IRGANOX (trademark) 1010)

(Epoxy Group-Containing Compound)

C-1: a copolymer of styrene and glycidyl methacrylate (manufactured by NOF Corporation: MARPROOF (trademark) G-0250SP)

(Bluing Agent)

BL-1: bluing agent (manufactured by Bayer AG: MACROLEX VIOLET (trademark) B)

Table 1

| | | | Example | | | | | | | | | | | | Comparative Example | | | | | | |
|---|---|---|---|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 1 | 2 | 3 | 4 | 5 | 6 | 7 |
| PC resin | Polycarbonate resin (A) Molecular weight: 23,900 | PC-1 | 100 | 100 | 100 | 100 | 100 | 100 | 100 | 100 | - | 100 | 100 | 100 | 100 | 100 | 100 | 100 | 100 | - | 100 |
| | Polycarbonate resin (A) Molecular weight: 22,200 | PC-2 | - | - | - | - | - | - | - | - | 100 | - | - | - | - | - | - | - | - | - | - |
| | Polycarbonate resin Molecular weight: 19,700 | PC-3 | - | - | - | - | - | - | - | - | - | - | - | - | - | - | - | - | - | 100 | - |

(continued)

| | | | Example | | | | | | | | | | | | Comparative Example | | | | | | |
|---|---|---|---|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 1 | 2 | 3 | 4 | 5 | 6 | 7 |
| Additive | Ultraviolet absorber (B1) | UV-1 | 0.22 | 0.37 | 0.54 | 0.72 | 0.9 | 0.9 | 0.9 | 0.9 | 0.9 | 1 | 1.2 | 1.35 | - | - | - | - | 0.05 | 0.9 | 1.45 |
| | Ultraviolet absorber (B6) | UV-2 | - | - | - | - | - | - | 0.1 | - | - | - | - | - | - | 0.22 | - | - | - | - | - |
| | Ultraviolet absorber (B6) | UV-3 | - | - | - | - | - | - | - | - | - | - | - | - | - | - | 0.22 | 2.4 | - | - | - |
| | Heat stabilizer (B2) | P-1 | 0.03 | 0.03 | 0.03 | 0.03 | 0.03 | 0.03 | - | 0.03 | 0.03 | 0.03 | 0.03 | 0.03 | 0.03 | 0.03 | 0.03 | 0.03 | 0.03 | 0.03 | 0.03 |
| | Heat stabilizer (B2) | P-2 | - | - | - | - | - | - | 0.02 | - | - | - | - | - | - | - | - | - | - | - | - |
| | Mold release agent (B3) | R-1 | 0.1 | - | 0.1 | 0.1 | 0.1 | - | - | 0.1 | 0.1 | 0.1 | 0.1 | 0.1 | 0.1 | 0.1 | 0.1 | 0.1 | 0.1 | 0.1 | 0.1 |
| | Mold release agent (B3) | R-2 | - | 0.2 | - | - | - | 0.1 | - | - | - | - | - | - | - | - | - | - | - | - | - |
| | Antioxidant (B4) | H-1 | - | - | - | - | - | - | - | 0.05 | 0.05 | - | - | - | - | - | - | - | - | 0.05 | - |
| | Antioxidant (B4) | H-2 | - | - | - | - | - | 0.03 | - | - | - | - | - | - | - | - | - | - | - | - | - |
| | Epoxy group-containing compound (B5) | C-1 | - | - | - | - | 0.0075 | - | - | 0.0075 | 0.0075 | - | - | - | - | - | - | - | - | 0.0075 | - |
| | Bluing agent (B7) | BL-1 | 0.00025 | 0.00025 | 0.00025 | 0.00025 | 0.00025 | 0.00025 | 0.00025 | 0.00025 | 0.00025 | 0.00025 | 0.00025 | 0.00025 | 0.00025 | 0.00025 | 0.00025 | 0.00025 | 0.00025 | 0.00025 | 0.00025 |

(continued)

| | | | Example | | | | | | | | | | | | Comparative Example | | | | | | |
|---|---|---|---|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 1 | 2 | 3 | 4 | 5 | 6 | 7 |
| Evaluation Results | Charpy impact strength (kJ/$m^2$) | | >80.0 | >80.0 | >80.0 | >80.0 | >80.0 | >80.0 | >80.0 | >80.0 | >80.0 | >70.0 | >60.0 | >55.0 | >80.0 | >80.0 | >80.0 | <55.0 | >80.0 | <55.0 | <55.0 |
| | Heat deflection temperature under load (°C) | | >125.0 | >125.0 | >125.0 | >125.0 | >125.0 | >125.0 | >125.0 | >125.0 | >125.0 | >125.0 | >124.0 | >124.0 | >125.0 | >125.0 | >125.0 | <124.0 | >125.0 | <124.0 | <124.0 |
| | Total light transmittance (%) | | >87.0 | >87.0 | >87.0 | >87.0 | >87.0 | >87.0 | >87.0 | >87.0 | >87.0 | >87.0 | >87.0 | >87.0 | >87.0 | >87.0 | >87.0 | <87.0 | >87.0 | >87.0 | >87.0 |
| | Spectral transmittance (%) | 420 nm | <70.0 | <70.0 | <70.0 | <70.0 | <70.0 | <70.0 | <70.0 | <70.0 | <70.0 | <70.0 | <70.0 | <70.0 | >70.0 | >70.0 | >70.0 | <70.0 | >70.0 | <70.0 | <70.0 |
| | | 400 nm | <1.0 | <1.0 | <1.0 | <1.0 | <1.0 | <1.0 | <1.0 | <1.0 | <1.0 | <1.0 | <1.0 | <1.0 | >80.0 | >70.0 | <1.0 | <1.0 | <1.0 | <1.0 | <1.0 |
| | Molding residence test (ΔE) | | <0.75 | <0.75 | <0.75 | <0.75 | <0.75 | <0.75 | <0.75 | <0.75 | <0.75 | <0.75 | <0.75 | <0.75 | <0.75 | <0.75 | <0.75 | >0.75 | <0.75 | <0.75 | >0.75 |

Table 2

| | | | Example | | | | | | | | | | | |
|---|---|---|---|---|---|---|---|---|---|---|---|---|---|---|
| | | | 13 | 14 | 15 | 16 | 17 | 18 | 19 | 20 | 21 | 22 | 23 | 24 |
| PC resin | Polycarbonate resin (A) molecular weight: 23,900 | PC-1 | 100 | 100 | 100 | 100 | 100 | 100 | 100 | 100 | - | 100 | 100 | 100 |
| | Polycarbonate resin (A) molecular weight: 22,200 | PC-2 | - | - | - | - | - | - | - | - | 100 | - | - | - |
| | Polycarbonate resin molecular weight: 19,700 | PC-3 | - | - | - | - | - | - | - | - | - | - | - | - |

(continued)

| | | | Example | | | | | | | | | | | |
|---|---|---|---|---|---|---|---|---|---|---|---|---|---|---|
| | | | 13 | 14 | 15 | 16 | 17 | 18 | 19 | 20 | 21 | 22 | 23 | 24 |
| Additive | Ultraviolet absorber (B1) | UV-4 | 0.12 | - | - | 0.50 | - | - | - | 0.90 | 0.90 | - | - | - |
| | Ultraviolet absorber (B1) | UV-5 | - | 0.37 | 0.45 | - | 0.90 | 0.90 | 0.90 | - | - | 1.00 | 1.20 | 1.35 |
| | Ultraviolet absorber (B6) | UV-6 | - | - | - | - | - | - | - | - | - | - | - | - |
| | Ultraviolet absorber (B6) | UV-2 | - | - | - | - | - | - | 0.1 | - | - | - | - | - |
| | Ultraviolet absorber (B6) | UV-3 | - | - | - | - | - | - | - | - | - | - | - | - |
| | Heat stabilizer (B2) | P-1 | 0.03 | 0.03 | 0.05 | 0.03 | 0.03 | 0.03 | - | 0.03 | 0.03 | 0.03 | 0.03 | 0.03 |
| | Heat stabilizer (B2) | P-2 | - | - | - | - | - | - | 0.02 | - | - | - | - | - |
| | Mold release agent (B3) | R-1 | - | - | 0.10 | 0.10 | 0.10 | 0.10 | - | 0.10 | 0.10 | 0.10 | 0.10 | 0.10 |
| | Mold release agent (B3) | R-2 | 0.20 | 0.20 | - | - | - | - | - | - | - | - | - | - |
| | Antioxidant (B4) | H-1 | - | - | - | - | - | 0.05 | - | 0.05 | 0.05 | - | - | - |
| | Antioxidant (B4) | H-2 | - | - | - | - | 0.03 | - | - | - | - | - | - | - |
| | Epoxy group-containing compound (B5) | C-1 | - | - | 0.0075 | 0.0075 | 0.0075 | 0.0075 | - | 0.0075 | 0.0075 | - | - | - |
| | Bluing agent (B7) | BL-1 | 0.0001 5 | 0.0002 5 | 0.0002 5 | 0.0002 5 | 0.0002 5 | 0.0002 5 | 0.0002 5 | 0.0002 5 | 0.0002 5 | 0.0002 5 | 0.0002 5 | 0.0002 5 |

(continued)

| | | | Example | | | | | | | | | | | |
|---|---|---|---|---|---|---|---|---|---|---|---|---|---|---|
| | | | 13 | 14 | 15 | 16 | 17 | 18 | 19 | 20 | 21 | 22 | 23 | 24 |
| Evaluation results | Charpy impact strength ($kJ/m^2$) | | >80.0 | >80.0 | >80.0 | >80.0 | >80.0 | >80.0 | >80.0 | >80.0 | >80.0 | >80.0 | >55.0 | >55.0 |
| | Heat deflection temperature under load (°C) | | >125.0 | >125.0 | >125.0 | >125.0 | >125.0 | >125.0 | >125.0 | >125.0 | >125.0 | >125.0 | >124.0 | >124.0 |
| | Total light transmittance (%) | | >87.0 | >87.0 | >87.0 | >87.0 | >87.0 | >87.0 | >87.0 | >87.0 | >87.0 | >87.0 | >87.0 | >87.0 |
| | Spectral transmittance (%) | 420 nm | <70.0 | <70.0 | <70.0 | <70.0 | <70.0 | <70.0 | <70.0 | <70.0 | <70.0 | <70.0 | <70.0 | <70.0 |
| | | 400 nm | <1.0 | <1.0 | <1.0 | <1.0 | <1.0 | <1.0 | <1.0 | <1.0 | <1.0 | <1.0 | <1.0 | <1.0 |
| | Molding residence test ($\Delta E$) | | <0.75 | <0.75 | <0.75 | <0.75 | <0.75 | <0.75 | <0.75 | <0.75 | <0.75 | <0.75 | <0.75 | <0.75 |
| | Dry heat resistance test ($\Delta YI$) | | <3.0 | <3.0 | <3.0 | <3.0 | <3.0 | <3.0 | <3.0 | <3.0 | <3.0 | <3.0 | <3.0 | <3.0 |

Table 3

| | | | Comparative Example | | | | | | | | |
|---|---|---|---|---|---|---|---|---|---|---|---|
| | | | 8 | 9 | 10 | 11 | 12 | 13 | 14 | 15 | 16 |
| PC resin | Polycarbonate resin (A) molecular weight: 23,900 | PC-1 | 100 | 100 | 100 | 100 | 100 | 100 | - | 100 | 100 |
| | Polycarbonate resin (A) molecular weight: 22,200 | PC-1 | - | - | - | - | - | - | - | - | - |
| | Polycarbonate resin molecular weight: 19,700 | PC-3 | - | - | - | - | - | - | 100 | - | - |
| Additive | Ultraviolet absorber (B1) | UV-4 | - | - | - | - | - | - | - | - | - |
| | Ultraviolet absorber (B1) | UV-5 | - | - | - | - | 0.05 | - | 0.90 | - | 1.5 |
| | Ultraviolet absorber (B6) | UV-6 | - | - | - | - | - | 0.37 | - | 0.90 | - |
| | Ultraviolet absorber (B6) | UV-2 | - | 0.22 | - | - | - | - | - | - | - |
| | Ultraviolet absorber (B6) | UV-3 | - | - | 0.22 | 2.4 | - | - | - | - | - |
| | Heat stabilizer (B2) | P-1 | 0.03 | 0.03 | 0.03 | 0.03 | 0.03 | 0.03 | 0.03 | 0.03 | 0.03 |
| | Heat stabilizer (B2) | P-2 | - | - | - | - | - | - | - | - | - |
| | Mold release agent (B3) | R-1 | 0.10 | 0.10 | 0.10 | 0.10 | 0.10 | - | 0.10 | 0.10 | 0.10 |
| | Mold release agent (B3) | R-2 | - | - | - | - | - | 0.20 | - | - | - |
| | Antioxidant (B4) | H-1 | - | - | - | - | - | - | 0.05 | 0.05 | - |
| | Antioxidant (B4) | H-2 | - | - | - | - | - | - | - | - | - |
| | Epoxy group-containing compound (B5) | C-1 | - | - | - | - | - | - | - | 0.0075 | - |
| | Bluing agent (B7) | BL-1 | 0.00025 | 0.00025 | 0.00025 | 0.00025 | 0.00025 | 0.00025 | 0.00025 | 0.00025 | 0.00025 |

(continued)

| | | | Comparative Example | | | | | | | | |
|---|---|---|---|---|---|---|---|---|---|---|---|
| | | | 8 | 9 | 10 | 11 | 12 | 13 | 14 | 15 | 16 |
| Evaluation results | Charpy impact strength (kJ/m$^2$) | | >80.0 | >80.0 | >80.0 | <55.0 | >80.0 | >80.0 | <55.0 | >80.0 | <55.0 |
| | Heat deflection temperature under load (°C) | | >128.0 | >128.0 | >128.0 | <124.0 | >128.0 | >128.0 | <124.0 | >125.0 | <124.0 |
| | Total light transmittance (%) | | >87.0 | >87.0 | >87.0 | <87.0 | >87.0 | >87.0 | >87.0 | >87.0 | >87.0 |
| | Spectral transmittance (%) | 420 nm | >70.0 | >70.0 | >70.0 | <70.0 | >70.0 | <70.0 | <70.0 | <70.0 | <70.0 |
| | | 400 nm | >80.0 | >70.0 | <1.0 | <1.0 | <1.0 | <1.0 | <1.0 | <1.0 | <1.0 |
| | Molding residence test (ΔE) | | <0.75 | <0.75 | <0.75 | >0.75 | <0.75 | <0.75 | <0.75 | <0.75 | >0.75 |
| | Dry heat resistance test (ΔYI) | | <3.0 | <3.0 | <3.0 | <3.0 | <3.0 | >3.0 | <3.0 | >3.0 | <3.0 |

<Results>

As seen from the results shown in Table 1, the polycarbonate resin compositions of Examples 1 to 24, which contained a prescribed amount of the specific ultraviolet absorber (B1), were confirmed to have an effect of reducing the light transmittance at not only 400 nm but also 420 nm in particular, as well as excellent impact resistance, molding residence stability, and dry heat resistance.

INDUSTRIAL APPLICABILITY

A high total light transmittance and excellent molding residence stability in a molding process or an extrusion process are properties of great importance for a polycarbonate resin composition as a molding material used in transparent plastic molded articles such as spectacle lenses. In addition, for example, when a molded article is used in a high-temperature environment such as the inside of an automobile during summertime, the molded article itself is required to have not only a heat resistance (deformation of the molded article under a high temperature is limited), but also a dry heat resistance (hue change and deterioration of the molded article are limited when the molded article is left to stand under a high temperature over a prolonged period).

The polycarbonate resin composition of the present invention maintains a high total light transmittance while reducing the transmittance of light having a wavelength of about 420 nm, particularly 400 to 420 nm, and exhibits excellent impact resistance, molding residence stability, and dry heat resistance; therefore, it can be widely used industrially as a molding material for obtaining hot-melt-molded articles in which these characteristics are utilized, such as front panels of various displays of personal computers, car navigation systems and the like, front panels and covers of light emitters, light source covers, multi-purpose sheets (e.g., injection-molded flat plates, and extrusion-molded flat plates and films), and spectacle lenses.

## Claims

1. A polycarbonate resin composition, comprising:

   a polycarbonate resin (A) having a viscosity-average molecular weight of 21,000 to 26,000; and
   with respect to 100 parts by mass of the polycarbonate resin (A), 0.1 to 1.5 parts by mass of an additive (B), and not less than 0.1 parts by mass of an ultraviolet absorber (B1) having a benzotriazole skeleton represented by the following Formula 1 as the additive (B):

   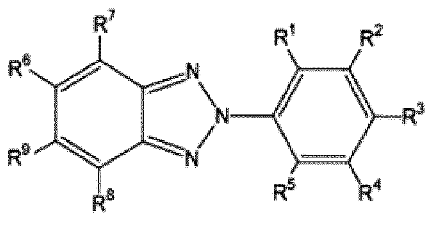

   Formula 1

   wherein,

   $R^1$ to $R^5$ each independently represent any of a hydrogen atom, a hydrocarbon group having 1 to 20 carbon atoms, and a hydroxy group, which hydrocarbon group optionally comprises an oxygen-containing group; and
   $R^6$ to $R^9$ each independently represent any of a hydrogen atom and a sulfur-containing group represented by R-S-, at least one of which $R^6$ to $R^9$ is a sulfur-containing group represented by R-S-, and which R is a hydrocarbon group having 1 to 24 carbon atoms, or an aromatic group having 6 to 24 carbon atoms in which a hydrogen atom is optionally substituted with an alkyl group having 1 to 18 carbon atoms.

2. The composition according to claim 1, wherein R of the sulfur-containing group represented by R-S- in the ultraviolet absorber (B1) is a hydrocarbon group having 1 to 24 carbon atoms.

3. The composition according to claim 2, comprising 0.2 to 1.5 parts by mass of the additive (B), and not less than 0.2 parts by mass of the ultraviolet absorber (B1), with respect to 100 parts by mass of the polycarbonate resin (A).

4. The composition according to claim 2 or 3, wherein R of the sulfur-containing group represented by R-S- in the ultraviolet absorber (B1) represents an alkyl group having 1 to 24 carbon atoms.

5. The composition according to claim 1, wherein R of the sulfur-containing group represented by R-S- in the ultraviolet absorber (B1) is an aromatic group having 6 to 24 carbon atoms in which a hydrogen atom is optionally substituted with an alkyl group having 1 to 18 carbon atoms.

6. The composition according to claim 5, wherein R of the sulfur-containing group represented by R-S- in the ultraviolet absorber (B1) represents a phenyl residue.

7. The composition according to any one of claims 1 to 6, wherein the sulfur-containing group represented by R-S- in the ultraviolet absorber (B1) is bound to $R^6$ or $R^9$.

8. The composition according to any one of claims 1 to 7, wherein the hydrocarbon group represented by $R^1$ to $R^5$ in the ultraviolet absorber (B1) is an alkyl group having 1 to 12 carbon atoms.

9. The composition according to any one of claims 1 to 8, wherein the hydrocarbon group represented by $R^1$ to $R^5$ in the ultraviolet absorber (B1) is an alkyl group having 1 to 4 carbon atoms.

10. The composition according to any one of claims 1 to 8, wherein at least one of $R^1$ to $R^5$ in the ultraviolet absorber (B1) is a methyl group.

11. The composition according to any one of claims 1 to 8, wherein at least one of $R^1$ to $R^5$ in the ultraviolet absorber (B1) is a methyl group, and at least one of the remaining $R^1$ to $R^5$ is a butyl group.

12. The composition according to any one of claims 1 to 11, comprising 0.1 to 1.4 parts by mass of the ultraviolet absorber (B1) with respect to 100 parts by mass of the polycarbonate resin (A).

13. The composition according to any one of claims 1 to 12, comprising 0.01 to 0.1 parts by mass of a phosphorus-based heat stabilizer (B2) as the additive (B) with respect to 100 parts by mass of the polycarbonate resin (A).

14. The composition according to any one of claims 1 to 13, comprising 0.03 to 0.5 parts by mass of a fatty acid ester-based mold release agent (B3) as the additive (B) with respect to 100 parts by mass of the polycarbonate resin (A).

15. The composition according to any one of claims 1 to 14, comprising 0.01 to 0.30 parts by mass of a hindered phenol-based antioxidant (B4) as the additive (B) with respect to 100 parts by mass of the polycarbonate resin (A).

16. The composition according to any one of claims 1 to 15, comprising 0.001 to 0.02 parts by mass of an epoxy group-containing compound (B5) as the additive (B) with respect to 100 parts by mass of the polycarbonate resin (A).

17. The composition according to any one of claims 1 to 16, comprising 0.05 to 0.3 parts by mass of an ultraviolet absorber (B6) other than the ultraviolet absorber (B1) as the additive (B) with respect to 100 parts by mass of the polycarbonate resin (A).

18. The composition according to any one of claims 1 to 17, which is used as a molding material of a hot-melt-molded article.

19. The composition according to any one of claims 1 to 17, which is used as a molding material of a spectacle lens molded article.

## INTERNATIONAL SEARCH REPORT

International application No.
PCT/JP2020/007514

A. CLASSIFICATION OF SUBJECT MATTER
C08K 5/101(2006.01)i; C08K 5/13(2006.01)i; C08K 5/3472(2006.01)i; C08K 5/3477(2006.01)i; C08K 5/521(2006.01)i; C08L 69/00(2006.01)i
FI: C08L69/00; C08K5/3477; C08K5/521; C08K5/101; C08K5/13; C08K5/3472
According to International Patent Classification (IPC) or to both national classification and IPC

B. FIELDS SEARCHED
Minimum documentation searched (classification system followed by classification symbols)
C08K5/101; C08K5/13; C08K5/3472; C08K5/3477; C08K5/521; C08L69/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | WO 2016/021664 A1 (MIYOSHI OIL & FAT CO., LTD.) 11.02.2016 (2016-02-11) | 1-19 |
| A | WO 2016/174788 A1 (TOKAI OPTICAL CO., LTD.) 03.11.2016 (2016-11-03) | 1-19 |
| A | JP 2017-132948 A (MIYOSHI OIL & FAT CO., LTD.) 03.08.2017 (2017-08-03) | 1-19 |
| A | JP 2018-122449 A (MITSUBISHI PAPER MILLS LIMITED) 09.08.2018 (2018-08-09) | 1-19 |
| A | JP 2017-201002 A (MITSUI CHEMICALS, INC.) 09.11.2017 (2017-11-09) | 1-19 |
| A | JP 2018-184519 A (MITSUI CHEMICALS, INC.) 22.11.2018 (2018-11-22) | 1-19 |
| A | JP 7-11138 A (PHILLIPS PETROLEUM COMPANY) 13.01.1995 (1995-01-13) | 1-19 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 April 2020 (01.04.2020) | 14 April 2020 (14.04.2020) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2020/007514

C (Continuation). DOCUMENTS CONSIDERED TO BE RELEVANT

| Category* | Citation of document, with indication, where appropriate, of the relevant passages | Relevant to claim No. |
|---|---|---|
| A | JP 2002-543266 A (CIBA SPECIALTY CHEMICALS HOLDING INC.) 17.12.2002 (2002-12-17) | 1-19 |

Form PCT/ISA/210 (continuation of second sheet) (January 2015)

**INTERNATIONAL SEARCH REPORT**
Information on patent family members

International application No.
PCT/JP2020/007514

| Patent Documents referred in the Report | Publication Date | Patent Family | Publication Date |
|---|---|---|---|
| WO 2016/021664 A1 | 11 Feb. 2016 | US 2017/0217937 A1<br>US 2019/0248766 A1<br>US 2019/0315721 A1<br>US 2019/0315722 A1<br>EP 3178898 A1<br>KR 10-2017-0041802 A<br>CN 106687550 A | |
| WO 2016/174788 A1 | 03 Nov. 2016 | US 2018/0134872 A1<br>EP 3290995 A1<br>CN 107533242 A<br>KR 10-2018-0020954 A | |
| JP 2017-132948 A | 03 Aug. 2017 | (Family: none) | |
| JP 2018-122449 A | 09 Aug. 2018 | (Family: none) | |
| JP 2017-201002 A | 09 Nov. 2017 | (Family: none) | |
| JP 2018-184519 A | 22 Nov. 2018 | (Family: none) | |
| JP 7-11138 A | 13 Jan. 1995 | US 5274015 A<br>US 5319091 A<br>US 5410071 A<br>US 5268450 A<br>EP 599269 A1<br>CA 2102477 A<br>KR 10-1994-0011582 A | |
| JP 2002-543266 A | 17 Dec. 2002 | JP 2011-51994 A<br>JP 2011-63803 A<br>US 6187845 B1<br>US 6268415 B1<br>US 2001/0011113 A1<br>US 6245915 B1<br>WO 2000/066676 A1<br>EP 1175467 A1<br>BR 10228 A<br>CZ 20013935 A<br>CA 2372686 A<br>AU 4554400 A<br>TW 249561 B<br>CN 1349552 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 5620033 B
- JP 4334633 B
- WO 2016021664 A
- WO 2016174788 A